(19) Europäisches Patentamt
European Patent Office
Office européen des brevets

(11) EP 1 202 120 A2

(12) EUROPEAN PATENT APPLICATION

(43) Date of publication:
02.05.2002 Bulletin 2002/18

(51) Int Cl.[7]: G03G 5/06

(21) Application number: 01308996.6

(22) Date of filing: 23.10.2001

(84) Designated Contracting States:
AT BE CH CY DE DK ES FI FR GB GR IE IT LI LU MC NL PT SE TR
Designated Extension States:
AL LT LV MK RO SI

(30) Priority: 23.10.2000 US 242517 P
08.06.2001 US 296803 P
08.06.2001 US 296806 P
08.06.2001 US 296822 P
08.06.2001 US 296979 P
06.07.2001 US 303567 P
06.07.2001 US 303631 P
10.08.2001 US 311644 P

(71) Applicant: SAMSUNG ELECTRONICS CO., LTD.
Suwon-City, Kyungki-do (KR)

(72) Inventors:
- Jubran, Nusrailah
St Paul, 55119 (US)
- Lee, Hwan-koo
Suwon-city, Kyungki-do (KR)
- Law, Kam W., Samsung Inf. Systems America
Woodbury, MN 55125 (US)

(74) Representative: Pidgeon, Robert John et al
Appleyard Lees
15 Clare Road
Halifax West Yorkshire HX1 2HY (GB)

# (54) Charge transport compound, elctrophotographic organophotoreceptor comprising the same, and electrographic imaging apparatus and method

(57) A charge transport compound having hydrazone-bridged heterocyclic groups connected by a central bridging group, and an organophotoreceptor including the same are provided. The charge transport compound has formula (1) below:

$$(R\text{-}Q)_n\text{-}Y \qquad (1)$$

where R is selected from the group consisting of julolidine ring groups, carbazole ring groups, and triarylmethane ring groups; Q is an aromatic hydrazone linking group having the following formula:

Z
|
=N-N-X-

where Z is an aromatic group, preferably a $C_6$ to $C_{20}$ aryl group, and X is a linking group, Y, a bridging group between R-Q- groups, is a chemical bond, carbon atom, nitrogen atom, oxygen atom, sulfur atom, a branched or linear-$(CH_2)_p$- group where p is an integer from 0 to 10, a $C_6$-$C_{20}$ aryl group, a $C_5$-$C_{20}$ cycloalkyl group, a cyclosiloxyl group, a heterocyclic group, or a $CR_{10}$ group where $R_{10}$ is hydrogen atom, a $C_1$-$C_{20}$ alkyl group, or a $C_6$-$C_{20}$ aryl group, and n is an integer from 2 to 6. The organophotoreceptor includes the charge transport compound, a charge generating compound, and a conductive substrate.

EP 1 202 120 A2

## Description

The present invention relates to organophotoreceptors for use in electrophotography, and more particularly, to a novel charge transport compound, a flexible organophotoreceptor including the novel charge transport compound, an electrophotographic imaging apparatus and method.

In electrophotography, organophotoreceptors are formed as a plate, disc, sheet, belt, or drum by depositing an insulating photoconductive element on a conductive substrate. Organophotoreceptors are imaged by uniformly electrostatically charging the surface of the photoconductive element and then exposing the charged surface to a light source. The light exposure selectively dissipates charges in the irradiated area of the photoconductive layer, thereby forming a pattern of charged and uncharged areas. A liquid or solid toner is then deposited in either the charged or uncharged areas to create a toner image on the surface of the photoconductive layer. The resulting visible toner image can be transferred to the surface of a suitable recording medium as paper. This imaging process is repeated several times.

The photoconductive element has a single- or multi-layered structure. A single-layered photoconductive element is formed by depositing a charge transport material and charge generating material on a conductive substrate in combination with a polymeric binder. A multi-layered photoconductive layer is formed by separately depositing a charge transport layer and a charge generating layer, each of which is selectively combined with a polymeric binder, on a conductive substrate.

The charge transport layer and the charge generating layer for photoconductive elements have two arrangements: a dual layered arrangement and inverted dual layered arrangement. In the dual layered arrangement, the charge generating layer is deposited on a conductive substrate, and the charge transport layer is formed on the charge generating layer. In the inverted dual layered arrangement, the sequence of deposition of the charge generating layer and the charge transport layer is inverted.

In both the single- and multi-layered photoconductive elements, the charge generating material functions to generate charge carriers (holes or electrons) upon exposure to light. The charge transport material functions to accept and transport these charge carriers in the charge transport layer in order to discharge charges on the surface of the photoconductive element.

To produce high quality images, particularly after multiple cycles of the imaging process, it is desirable to form a homogeneous solution of charge transport material by mixing with a polymeric binder and remain in solution. In addition, it is desirable to maximize the charging capacity of the charge transport material (indicated by a parameter known as the acceptance voltage or "$V_{acc}$"), and to minimize charge retention by the same upon discharge (indicated by a parameter known as the residual voltage or "$V_{res}$").

There are many charge transport materials available for electrophotography: pyrazoline derivatives, fluorene derivatives, oxadiazole derivatives, stilbene derivatives, hydrazone derivatives, carbazole hydrazone derivatives, polyvinyl carbazole, polyvinyl pyrene, polyacenaphthylene, etc. However, all the charge transport materials listed above suffer some disadvantages. Therefore, a need exists for novel charge transport materials to meet the various requirements of electrophotography applications.

A first object of the present invention is to provide a novel charge transport compound capable of reducing or solving the above problems.

A second object of the present invention is to provide an organophotoreceptor with excellent mechanical and electrostatic characteristics by including the novel charge transport compound.

Third and fourth objects of the present invention are to provide an electrophotographic imaging apparatus and method capable of producing high quality images even after repeated imaging cycles, by adopting the organophotoreceeptor.

To achieve the first object of the present invention, there is provided a charge transport compound having formula (1) below:

$$(R\text{-}Q)_n\text{-}Y \qquad (1)$$

where R is selected from the group consisting of julolidine ring groups, carbazole ring groups, and triarylmethane ring groups, Q is an aromatic hydrazone linking group, Y is a bridging group or chemical bond between R-Q- groups, and n is an integer from 2 to 6.

Preferably, Q comprises an aromatic linking group having the following formula:

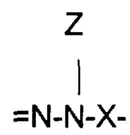

where Z is an aromatic group, especially $C_6$ to $C_{20}$ aryl, and X is a linking group. More preferably, Z is a phenyl group, X is $-(CH_2)_m-$, where m is an integer from 0 to 20. More preferably, X is an alkylene group. In this case, at least one methylene group in the alkylene group may be substituted with an oxygen atom, a carbonyl group, urethane, urea, an ester group, a $NR_6$ group, a $CHR_7$ group, or a $CR_8R_9$ group, where $R_6$, $R_7$, $R_8$, and $R_9$ are, independently, H, a $C_6$-$C_{20}$ alkyl group, or a $C_6$-$C_{20}$ aryl group.

In formula (1) above, it is preferable that Y is a chemical bond, carbon atom, nitrogen atom, oxygen atom, sulfur atom, a branched or linear $-(CH_2)_p-$ group where p is an integer from 0 to 10, a $C_6$-$C_{20}$ aryl group, a $C_5$-$C_{20}$ cycloalkyl group, a cyclosiloxyl group, a heterocyclic group, or a $CR_{10}$ group where $R_{10}$ is hydrogen atom, a $C_1$-$C_{20}$ alkyl group, or a $C_6$-$C_{20}$ aryl group.

It is preferable that the charge transport compound having formula (1) above according to the present invention is a compound having formula (2) below:

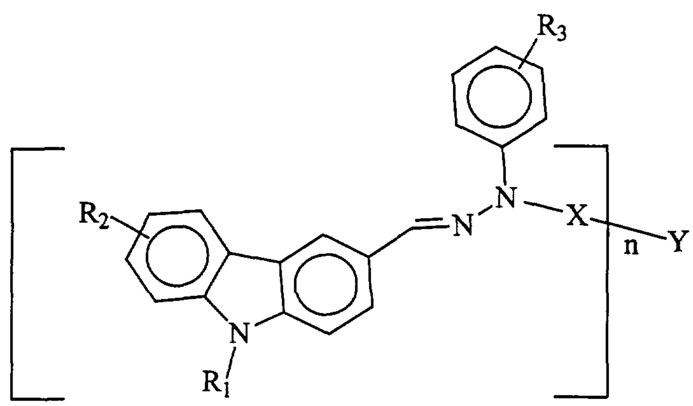

...(2)

where

n is an integer from 2 to 6;

$R_1$ is hydrogen, a branched or linear $C_1$-$C_{20}$ alkyl group, a branched or linear unsaturated $C_2$-$C_{20}$ hydrocarbon group, an ether group, or a $C_6$-$C_{20}$ aryl group;

$R_2$ is hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a $C_5$-$C_{20}$ cycloalkyl group, an aryl group, or a $-NR_4R_5$ group, where $R_4$ and $R_5$ are, independently, hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, a $C_6$-$C_{20}$ aryl group, or $R_4$ and $R_5$ form a ring in combination with nitrogen atom;

$R_3$ is hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a $C_5$-$C_{20}$ cycloalkyl group, or a $C_6$-$C_{20}$ aryl group;

X is a linking group having branched or linear $-(CH_2)_m-$, where m is an integer from 0 to 20, and at least one methylene groups is optionally substituted with oxygen atom, a carbonyl group, a urethane group, a urea group, an ester group, a $-NR_6$ group, a $CHR_7$ group, or a $CR_8R_9$ group where $R_6$, $R_7$, $R_8$, and $R_9$ are, independently, H, a $C_1$-$C_{20}$ alkyl group, or a $C_6$-$C_{20}$ aryl group; and

Y is a chemical bond, carbon atom, nitrogen atom, oxygen atom, sulfur atom, a branched or linear $-(CH_2)_p-$ group where p is an integer from 0 to 10, a $C_5$-$C_{20}$ aryl group, a $C_5$-$C_{20}$ cycloalkyl group, a cyclosiloxyl group, a heterocyclic group, or a $CR_{10}$ group where $R_{10}$ is hydrogen atom, a $C_1$-$C_{20}$ alkyl group, or a $C_6$-$C_{20}$ aryl group.

Preferably, the compound having formula (2) above includes compounds (A) through (F) having one of the

following formulae:

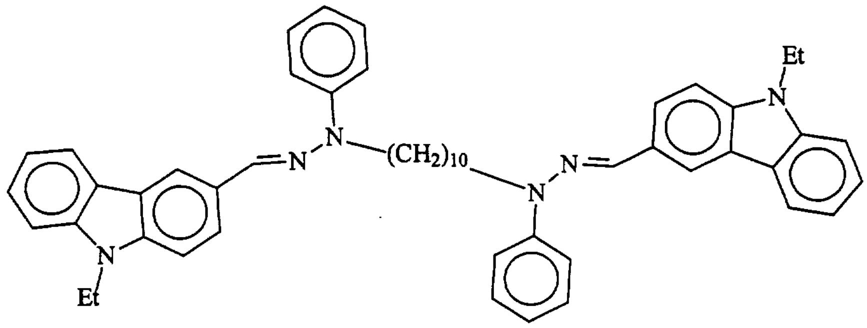

(A)

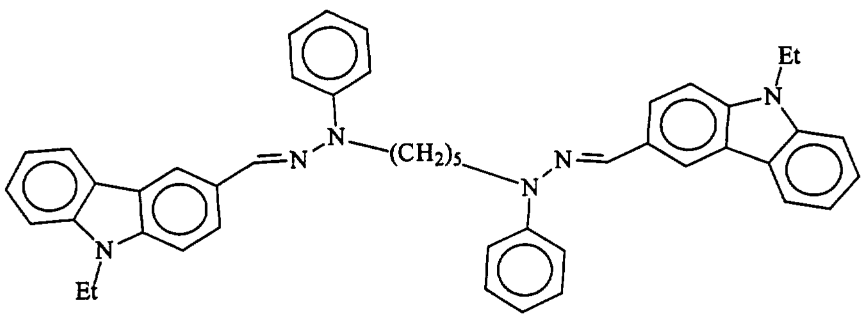

(B)

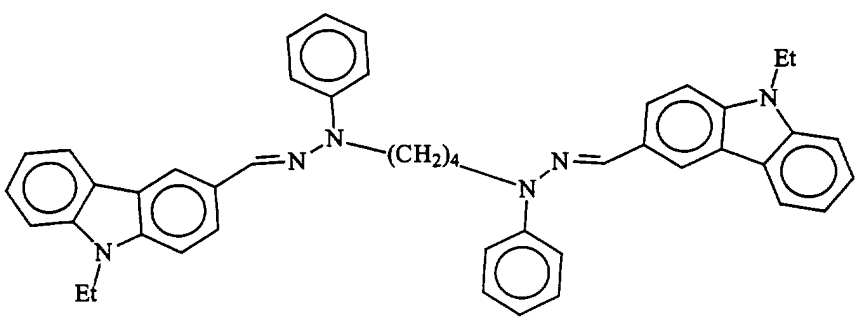

(C)

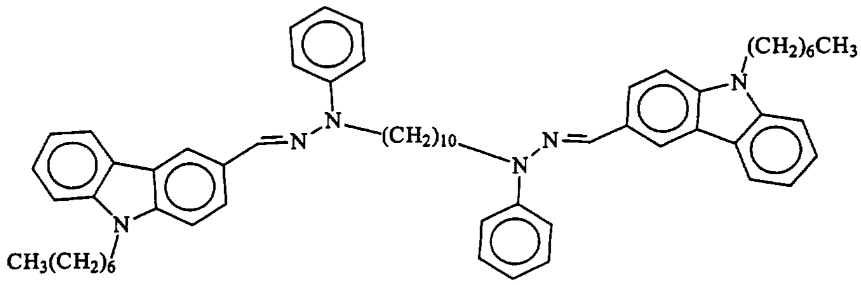

(D)

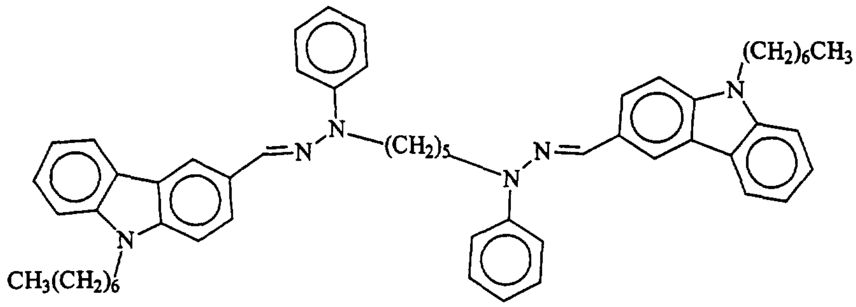

(E)

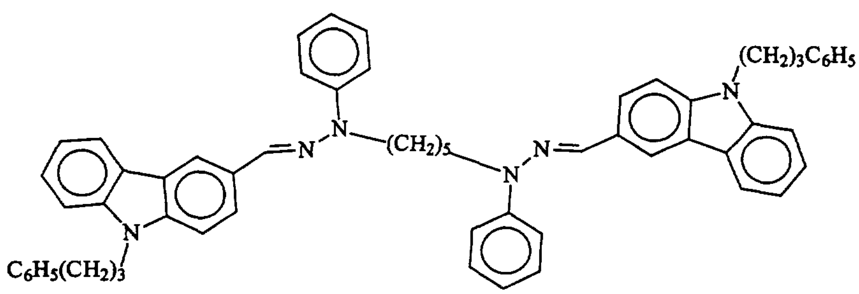

(F)

Preferably, the charge transport compound having formula (1) above according to the present invention may be a compound having formula (3):

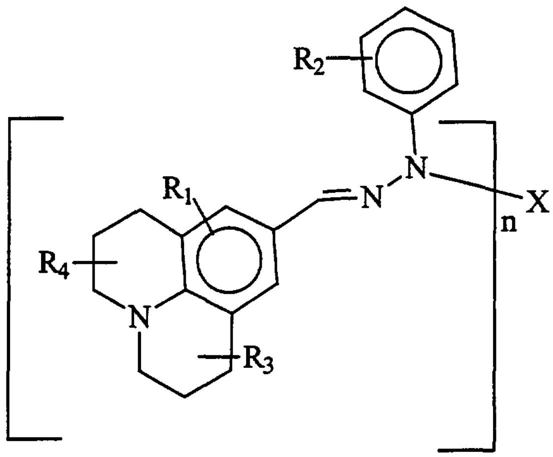

...(3)

where

n is an integer from 2 to 6;

$R_1$, $R_2$, $R_3$, and $R_4$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and

X is a linking group having branched or linear $-(CH_2)_m-$, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a $C_5$-$C_{20}$ cycloalkyl group, a cyclosiloxyl group, a heterocyclic group, a $NR_5$ group, a $CHR_6$ group, or a $CR_7R_8$ group where $R_5$, $R_6$, $R_7$, and $R_8$ are, independently, H, a $C_1$-$C_{20}$ alkyl group, or a $C_6$-$C_{20}$ aryl group.

More preferably, the compound having formula (3) above is a compound having formula (4) below:

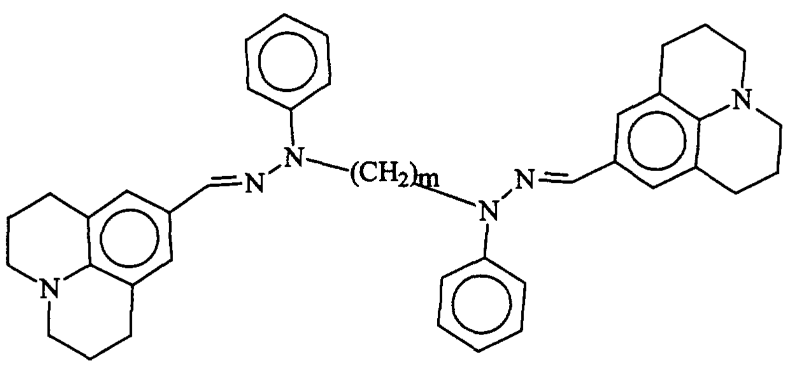

...(4)

where m is an integer from 4 to 10.

The charge transport compound having formula (1) above according to the present invention also includes a compound having formula (5) below:

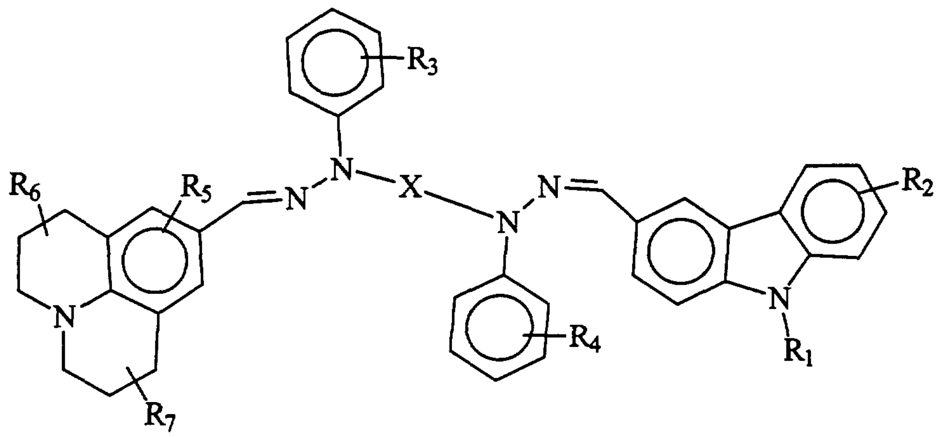

...(5)

where

$R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group;
$R_2$, $R_3$, $R_4$, $R_5$, $R_6$, and $R_7$ are, independently, hydrogen, halogen atom, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and
X is a linking group having a branched or linear $-(CH_2)_m-$, where m is an integer from 0 to 50, and at least one methylene group is optionally substituted with oxygen atom, sulfur atom, a carbonyl group, an urethane group, an urea group, an ester group, a $C_6$-$C_{20}$ aryl group, a heterocyclic group, a $C_5$-$C_{20}$ cycloalkyl group, a cyclosiloxyl group, a $NR_8$ group, a $CHR_9$ group, or a $CR_{10}R_{11}$ group where $R_8$, $R_9$, $R_{10}$, and $R_{11}$, are, independently, H, a $C_1$-$C_{20}$ alkyl group, or a $C_6$-$C_{20}$ aryl group.

The compound having formula (5) above may be compound (J) having the following formula:

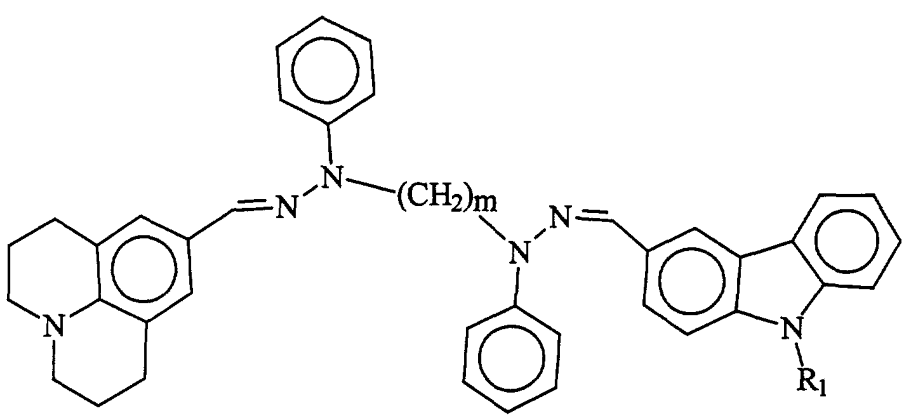

(J)

where m is an integer from 2 to 20, and $R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group.

Preferably, the charge transport compound having formula (1) above according to the present invention is a compound having formula (6) below:

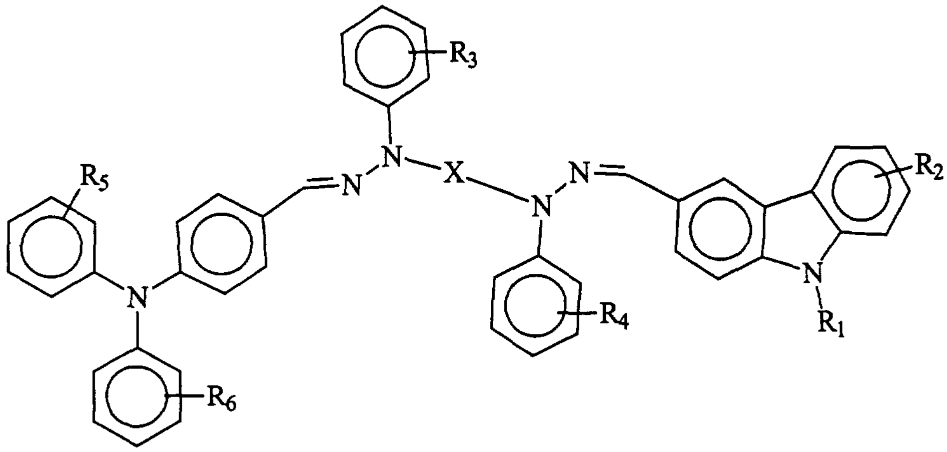

...(6)

where

$R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group;
$R_2$, $R_3$, $R_4$, $R_5$, and $R_6$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and
X is a linking group having branched or linear $-(CH_2)_m-$, where m is an integer from 0 to 50, and at least one methylene group is optionally substituted with oxygen atom, sulfur atom, a carbonyl group, an urethane group, an urea group, an ester group, a $C_6$-$C_{20}$ aryl group, a heterocyclic group, a $C_5$-$C_{20}$ cycloalkyl group, a cyclosiloxyl group, a $NR_7$ group, a $CHR_8$ group, or a $CR_9R_{10}$ group where $R_7$, $R_8$, $R_9$, and $R_{10}$, are, independently, H, a $C_1$-$C_{20}$ alkyl group, or a $C_6$-$C_{20}$ aryl group.

The compound having formula (6) above may be compound (K) having the following formula:

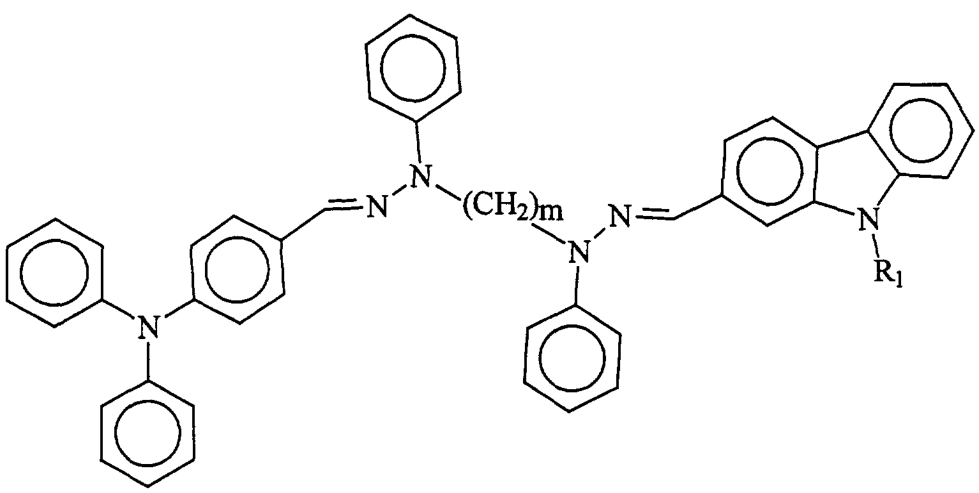

(K)

where m is an integer from 2 to 20, and $R_1$ is hydrogen, a branched or linear $C_1$-$C_{20}$ alkyl group, a branched or linear

unsaturated $C_2$-$C_{20}$ hydrocarbon group, a $C_5$-$C_{20}$ cycloalkyl group, or a $C_6$-$C_{20}$ aryl group;

Preferably, the charge transport compound having formula (1) above according to the present invention includes a compound having formula (7) below:

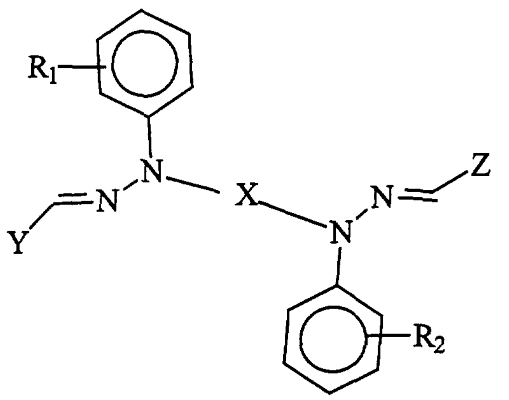

...(7)

wherein

R$_1$ and R$_2$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group;
X is a linking group having branched or linear $-(CH_2)_m-$, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a heterocyclic group, a $NR_3$ group, a $CHR_4$ group, or a $CR_5R_6$ group where $R_3$ , $R_4$, $R_5$, and $R_6$ are, independently, H, a $C_1$-$C_{20}$ alkyl group, or a $C_6$-$C_{20}$ aryl group; and
Y and Z are, independently, a carbazole group, a triphenylamine group, a julolidine group, or any of their derivatives.

A typical example of the compound having formula (7) above has formula (8) below:

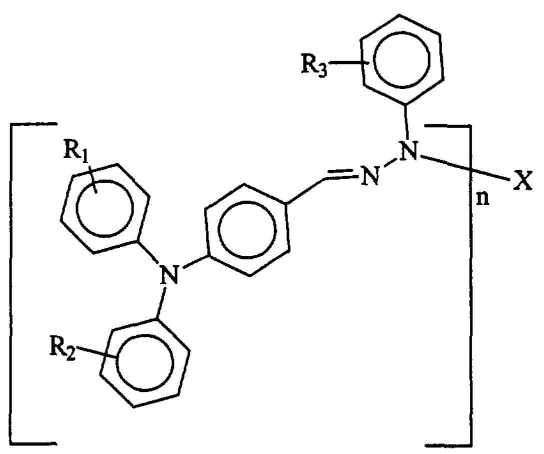

...(8)

where

n is an integer from 2 to 6;
$R_1$, $R_2$, and $R_3$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an

ether group, a nitro group, an amino group, a $C_5$-$C_{20}$ cycloalkyl group, or a $C_6$-$C_{20}$ aryl group; and
X is a linking group having branched or linear -$(CH_2)_m$-, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a heterocyclic group, a $NR_4$ group, a $CHR_5$ group, or a $CR_6R_7$ group where $R_4$, $R_5$, $R_6$, and $R_7$ are, independently, H, a $C_1$-$C_{20}$ alkyl group, or a $C_6$-$C_{20}$ aryl group.

Preferably, the compound having formula (8) above is compound (M) having the following formula:

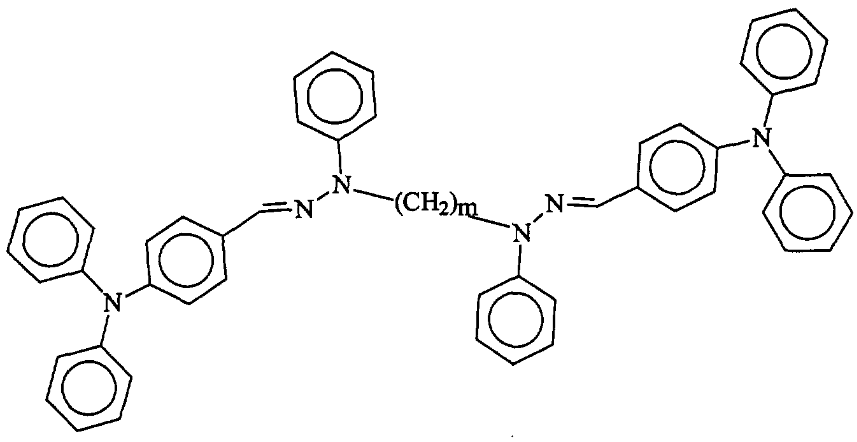

(M)

where m is an integer from 4 to 10.

Preferably, the charge transport compound having formula (1) above according to the present invention includes a compound having formula (9) below:

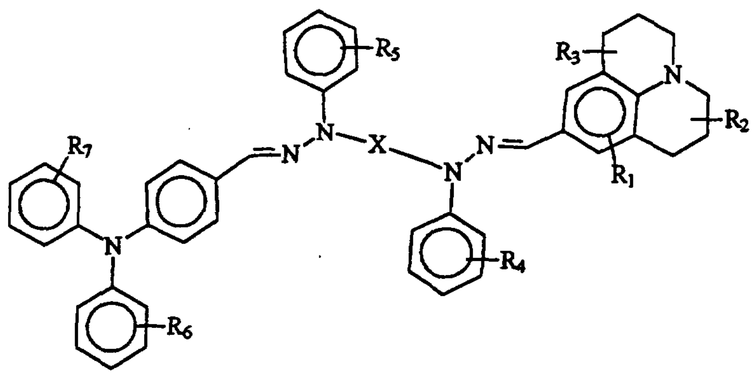

... (9)

where

$R_1$, $R_2$, $R_3$, $R_4$, $R_5$, $R_6$, and $R_7$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and
X is a linking group having branched or linear -$(CH_2)_m$-, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a heterocyclic group, a $NR_8$ group, a $CHR_9$ group, or a $CR_{10}R_{11}$ group where $R_8$, $R_9$, $R_{10}$, and $R_{11}$ are, independently, H, a $C_1$-$C_{20}$ alkyl group, or a $C_6$-$C_{20}$ aryl group.

Preferably, the compound having formula (9) above is compound (N) having the following formula:

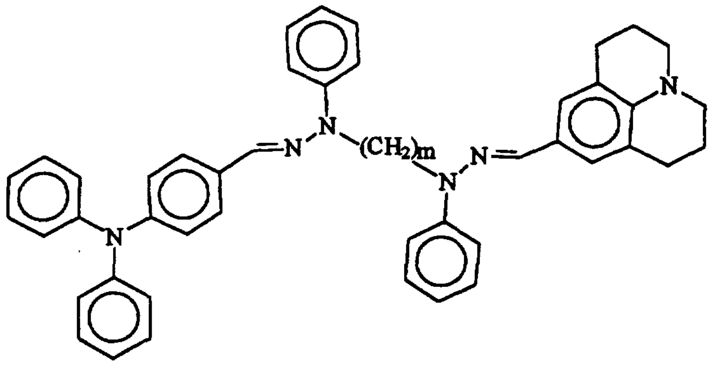

(N)

where m is an integer from 4 to 20.

Preferably, the charge transport compound having formula (1) above according to the present invention includes a compound having formula (10) below:

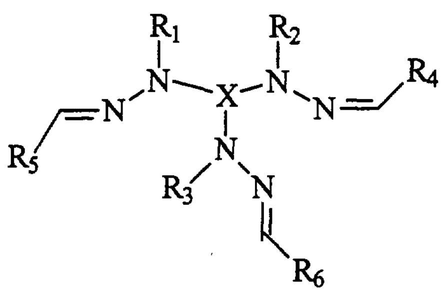

...(10)

where

$R_1$, $R_2$, and $R_3$ are, independently, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, a heterocyclic group, or a $C_6$-$C_{20}$ aryl group;

$R_4$, $R_5$, and $R_6$ are, independently, a carbocyclic group selected from the group consisting of triarylamine, diaryl alkylamine, dialkyl arylamine, anthraquinone, diphenoquinone, indane, and fluorenone, or a heterocyclic ring selected from the group consisting of thiazoline, thiazolidine, phenothiazine, oxazoline, imidazoline, imidazolidine, thiazole, oxazole, isoxazole, oxazolidinone, morpholine, imidazole, benzothiazole, benzotriazole, benzoxazole, benzimidazole, naphthothiazole, naphthoxazole, naphthimidazole, quinoline, isoquinoline, quinoxaline, indole, indazole, pyrrole, purine, pyrrolidine, pyridine, piperidine, pyridazine, pyrazoline, pyrimidine, pyrazine, triazole, oxadiazole, tetrazole, urazole, carbazole, julolidine, and thiadiazole, wherein the heterocyclic ring optionally have at least one substituent selected from the group consisting of halogen atom, halide, a $C_1$-$C_{20}$ alkyl group, a $C_1$-$C_{20}$ alkoxy group, a $C_6$-$C_{20}$ aryl group, a $C_6$-$C_{20}$ aryloxy group, a N-substituted amino group, a N-disubstituted amino group, acyl, carbamoyl, sulfamoyl, nitro, cyano, hydroxy, carboxy, sulfonate, oxo, benzo, naptho, indeno, and phosphate; and

X is a linking group having the following formula:

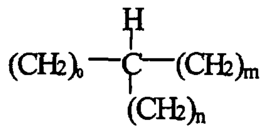

where m, n, and o is an integer from 0 and 50; at least one methylene group is substituted with a chemical bond, oxygen atom, sulfur atom, a carbonyl group, an urethane group, an urea group, an ester group, a $C_6$-$C_{20}$ aryl group, a heterocyclic group, a $NR_5$ group, a $CHR_6$ group, or a $CR_7R_8$ group where $R_5$ , $R_6$, $R_7$, and $R_8$ are, independently, H, a $C_1$-$C_{20}$ alkyl group, or a $C_6$-$C_{20}$ aryl group; and the C-H group is optionally substituted by nitrogen atom, boron atom, metal atom, and a $CR_9$ group where $R_9$ is a $C_1$-$C_{20}$ alkyl group or a $C_6$-$C_{20}$ aryl group.

Preferably, the compound having formula (10) above is compound (O) or (P) having the following formulae:

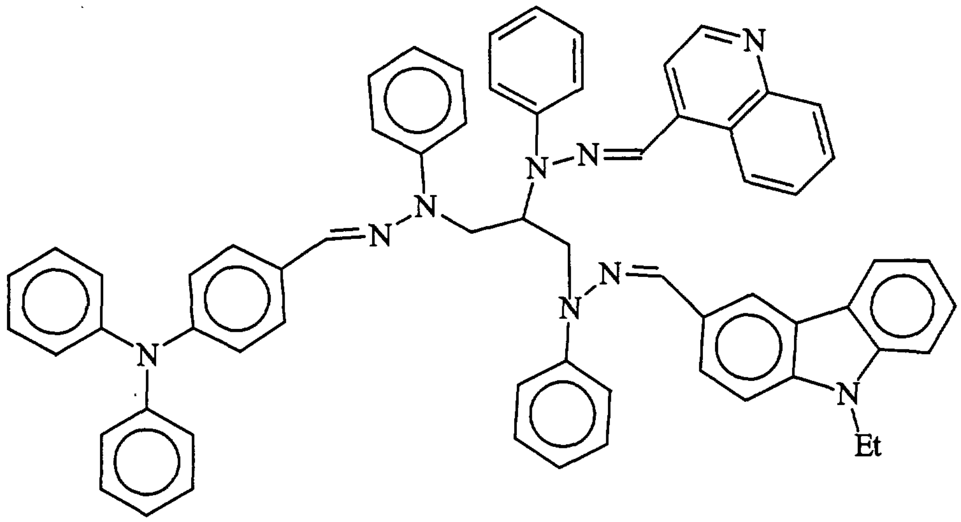

(O)

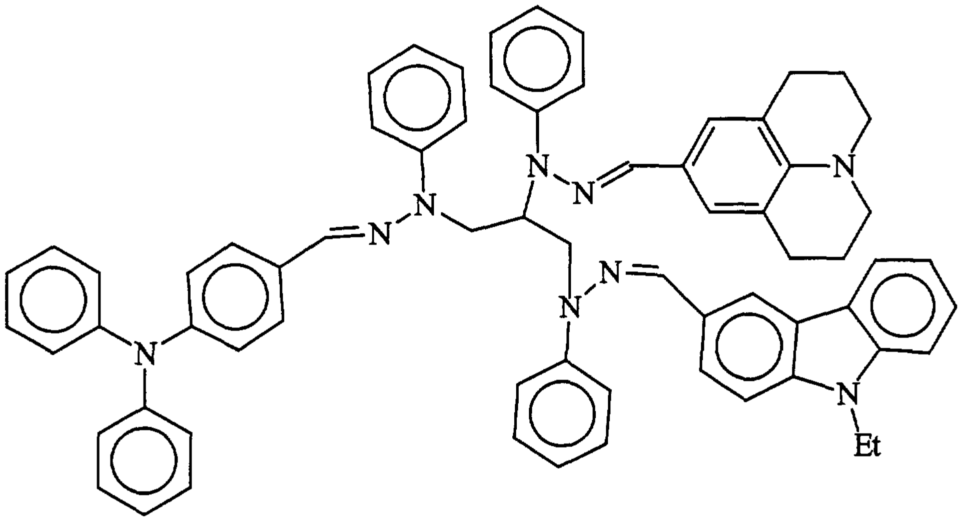

(P)

The second object of the present invention is achieved by an organophotoreceptor comprising a charge transport compound having formula (1) below, a charge generating compound, and a conductive substrate.

The third object of the present invention is achieved by an electrophotographic imaging apparatus comprising: a plurality of support rollers; and an organophotoreceptor in the form of a flexible belt wound around the support rollers, the organophotoreceptor including a charge transport compound having formula (1) below, a charge generating compound, and a conductive substrate. At least one of the support rollers preferably has a diameter no greater than 40 nm.

The fourth object of the present invention is achieved by an electropotographic imaging method involving: applying an electric charge to a surface of an organophotoreceptor including a charge transport compound having formula (1) below, a charge generating compound, and a conductive substrate; exposing the surface of the organophotoreceptor to radiation to dissipate charge in selected areas and thereby form a pattern of charged and uncharged areas on the surface; contacting the surface with a liquid toner comprising a dispersion of colorant particles in an organic liquid to create a toner image; and transferring the toner image to a substrate.

In describing chemicals by structural formulae and group definitions, certain terms are used in a nomenclature format that is chemically acceptable. The terms "group", "central nucleus", and "moiety" have defined meanings. The term "group" indicates that the generically recited chemical material (e.g., alkyl group, phenyl group, carbazole group, etc.) may have any substituent thereon which is consistent with the bond structure of that group. For example, alkyl group includes alkyl materials such as methyl ethyl, propyl isooctyl, dodecyl and the like, and also includes such substituted alkyls as chloromethyl, dibromoethyl, 1,3-dicyanopropyl, 1,3,5-trihydroxyhexyl, 1,3,5-trifluorocyclohexyl, 1-methoxy-dodecyl, and the like. However, as is consistent with such nomenclature, no substitution would be included within the term that would alter the fundamental bond structure of the basic group. For example, where a pheny ring group or central nucleus of a phenyl group is recited, substitututes such as 1-hydroxyphenyl, 2,4-fluorophenyl, orthocyanophenyl, 1,3,5-trimethoxyphenyl and the like would be acceptable within the terminology, while 1,1,2,2,3,3-hexamethylphenyl substitute would not be acceptable as that substitutent would require the ring bond structure of the phenyl group to be altered to a non-aromatic form. Similarly, where the term "central nucleus of the formula" is used and a structural formula is shown, any substituent may be provided on that formula, as long as the substitution does not alter the basic bond structure of the formula (e.g., by requiring a double bond to be converted to a single bond, or opening a ring group, or dropping a substituent in the formula). Where the term "moiety" is used, such as alkyl moiety or phenyl moiety, that terminology indicates that the chemical material is not substituted.

In general preferred aromatic groups herein are naphthyl and, especially, phenyl. Preferred alkyl groups are $C_{1-8}$ alkyl, especially $C_{1-4}$ alkyl. Preferred alkoxy groups are $C_{1-8}$ alkoxy, especially $C_{1-4}$ alkoxy. Preferred alkylene groups are $C_{1-10}$ alkylene, especially $C_{1-4}$ alkylene. Preferred amino is $-NH_2$. Preferred cycloalkyl groups are $C_{5-10}$ cycloalkyl. Preferred halogen atoms are bromine, fluorine and chlorine.

An organophotoreceptor according to the present invention is formed of the charge transport compound having formula (1) above, a charge generating compound, and a conductive substrate.

The organophotoreceptor may be in the form of a plate, a disc, a flexible belt, a rigid drum, or a sheet around a rigid or compliant drum, with the flexible belt being preferred. The organophotoreceptor may include a charge transport layer including the charge transport compound having formula (1) above and a polymeric binder, a charge generating layer including a charge generating compound and a polymeric binder, and a conductive substrate. The charge transport layer may be located between the charge generating layer and the conductive substrate. Alternatively, the charge generating layer may be interposed between the charge transport layer and the conductive substrate.

The present invention also provides an electrophotographic imaging apparatus including a plurality of support rollers; and the organophotoreceptor in the form of a flexible belt wound around the support rollers. In the electrophotographic imaging apparatus, preferably, at least one of the support rollers has a diameter no greater than 40 nm. The electrophotographic imaging apparatus according to the present invention may further include a liquid toner dispenser.

The present invention also provides an electrophotographic imaging method characterized by involving: applying an electric charge to a surface of the organophotoreceptor described above; imagewise exposing the surface of the organophotoreceptor to radiation to dissipate charge in selected areas and thereby form a pattern of charged and uncharged areas on the surface; contacting the surface with a liquid toner comprising a dispersion of colorant particles in an organic liquid to create a toner image; and transferring the toner image to a substrate.

The charge transport compound having formula (1) according to the present invention can be prepared by a multi-step synthesis using a combination of known synthetic techniques.

The first step is the preparation of one or more of aldehyde derivative of any heterocyclic compound by Vilsmeier reaction with phosphorus oxychloride ($POCl_3$).

Preferably, the heterocyclic compound includes carbazole, triphenylamine, julolidine, and triarylmethane ring groups. Other heterocyclic compounds may include thiazoline, thiazolidine, phenothiazine, oxazoline, imidazoline, imidazolidine, thiazole, oxazole, isoxazole, oxazolidinone, morpholine, imidazole, benzothiazole, benzotriazole, benzoxazole, benzimidazole, naphthothiazole, naphthoxazole, naphthimidazole, quinoline (e.g., 2-quinoline or 4-quinoline), isoquinoline, quinoxaline, indole, indazole, pyrrole, purine, pyrrolidine, pyridine, piperidine, pyridazine, pyrazoline, pyrimidine, pyrazine, triazole, oxadiazole, tetrazole, urazole, carbazole, and thiadiazole rings.

The second step is the reaction between phenylhydrazine and one of the aldehyde derivatives of carbazole,

triphenylamine, and julolidine in a molar ratio of 1:1 to form the corresponding hydrazone derivative by refluxing the reactants in THF for two hours. More than one hydrazone derivative may be prepared if an unsymmetrical charge transport compound is desired.

The last step is the reaction of hydrazone with dibromoalkane in a molar ratio of 2:1 to form a symmetrical charge transport compound. The hydrazone obtained is dissolved in DMSO. After the addition of 25% aqueous solution of NaOH, a dibromoalkane is added to the solution. This solution is stirred at 70°C for approximately 1 hour. The product from this reaction is purified by recrystallization. If an unsymmetrical charge transport compound is desired, two or more different hydrazones are used. Each hydrazone will react, one at a time, with a dibromoalkane or an alkane with more than two bromo groups in a molar ratio of 1:1 under the conditions described above.

Compound (M) may be prepared by the condensation reaction of 4-(diphenylamino)-benzaldehyde with phenyl hydrazine; and then by a nucleophilic substitution reaction of the condensation product with a dibromoalkane to form the final dimeric charge transport material. Specifically, compound (M) with m=5 may be prepared according to the above synthesis by using 1,5- dibromopentane as a dibromoalkane.

The invention provides novel charge transport compounds for organophotoreceptors featuring a combination of good mechanical and electrostatic properties. The photoreceptor formed of the novel charge transport compound can be used successfully with liquid toners to produce high quality images. The quality of the images is maintained after repeated imaging cycling.

Other features and advantages of the invention will be apparent from the following description of the preferred embodiments thereof and from the claims.

The invention features organic photoreceptors that include the charge transport compound having formula (1) set forth above. The charge transport compound having formula (1) may be prepared by a multi-step synthesis using a combination of known synthetic techniques. For example, compounds (E) and (F) and the compound having formula (4) with m=5 are prepared by a 4-step synthetic method.

The first step is N-alkylatation of carbazole to introduce an alkyl group to the carbazole nitrogen. The second step is the formation of a -CHO group on the carbazole ring by the Vilsmeier reaction. The third step is the formation of hydrazone by reaction of the product from the second step with a hydrazine. The last step is a nucleophilic substitution reaction to form a bridging group between two or more hydrazone moieties.

Compounds (B) through (D) are prepared by the method described above, except that the first and second steps were skipped because the starting materials for the third step are commercially available.

The organophotoreceptor according to the present invention may be in the form of a plate, a drum, or a belt, with the flexible belt being preferred. The organophotoreceptor may include a conductive substrate and a photoconductive element in the form of a single layer. The single-layered photoconductive element contains the charge transport compound having formula (1) above, a charge generating compound, and a polymeric binder. Preferably, the organophotoreceptor includes a conductive substrate, and a dual-layered photoconductive element having separate charge generating layer and charge transport layer. In this case, the charge generating layer is located between the charge transport layer and the conductive substrate. Alternatively, the charge transport layer may be interposed between the charge generating layer and the conductive substrate, thereby to form an inverted structure of the photoconductive element.

The electrically conductive substrate may be flexible, for example in the form of a flexible web or belt, or inflexible, for example in the form of a drum. Typically, the conductive substrate comprises an insulating substrate and a conductive thin film. The insulating substrate may be paper or a film-forming polymer such as polyethylene terephthalate, polyimide, polysulfone, polyethylene naphthalate, polypropylene, nylon, polyester, polycarbonate, polyvinyl fluoride, polystyrene and the like. Examples of the insulating substrate include polyethersulfone (Stabar S-100, available from ICI), polyvinyl fluoride (Tedlar, available from E.I. DuPont de Nemours & Company), polybisphenol-A polycarbonate (Makrofol, available from Mobay Chemical Company) and amorphous polyethylene terephthalate (Melinar, available from ICI Americas, Inc.). Conductive materials for the conductive thin film may include graphite, dispersed carbon black, iodide, conductive polymers such as polypyrroles and Calgon Conductive polymer 261 (available from Calgon Corporation, Inc., Pittsburgh, Pa.), metals such as aluminum, titanium, chromium, brass, gold, copper, palladium, nickel, or stainless steel, or metal oxides such as tin oxide or indium oxide. Preferably, the conductive material is aluminum. Typically, photoconductive substrates should be thick enough to satisfy the requirement for mechanical stability. For example, flexible web type substrates generally have a thickness of 0.01-1 mm, and drum type substrates generally have a thickness of 0.5-2 mm. Typical structures for polycarbonates are as follows:

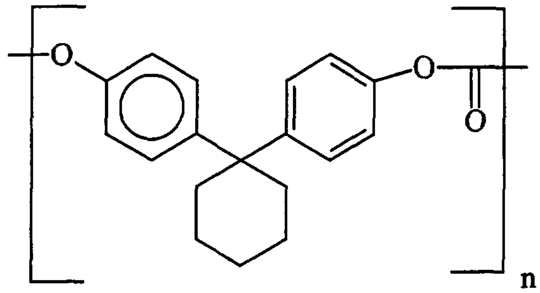

Polycarbonate Z

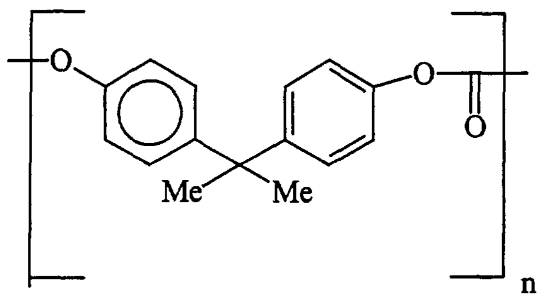

Polycarbonate A

The charge generating compound is a material which is capable of absorbing light to generate charge carriers, such as a dye or pigment. Examples of suitable charge generating compounds include metal-free phthalocyanines (e. g., Progen 1 x-form metal-free phthalocyanine from Zeneca, Inc.), metal phthalocyanines such as titanium phthalocyanine, copper phthalocyanine, oxytitanium phthalocyanine, hydroxygallium phthalocyanine, squarylium dyes and pigments, hydroxy-substituted squarylium pigments, perylimides, polynuclear quinones (Indofast Double Scarlet, Indofast Violet Lake B, Indofast Brilliant Scarlet, and Indofast Orange, available from Allied Chemical Corporation), quinacridones (Monastral Red, Monastral Violet, and Monastral Red Y, available from DuPont), naphthalene 1,4,5,8-tetracarboxylic acid derived pigments including perinones, tetrabenzoporphyrins, tetranaphthaloporphyrins, indigo- and thioindigo dyes, benzothioxanthene derivatives, perylene 3,4,9,10-tetracarboxylic acid derived pigments, polyazo-pigments including bisazo-, trisazo-, and tetrakisazo-pigments, polymethine dyes, dyes containing quinazoline groups, tertiary amines, amorphous selenium, selenium alloys such as selenium-tellurium, selenium-tellurium-arsenic, selenium-arsenic, cadmium sulphoselenide, and cadmiumselenide, cadmium sulphide, and mixtures of these materials. Preferably, the charge generating compound is oxytitanium phthalocyanine, hydroxygallium phthalocyanine, or a combination of these materials.

Preferably, the charge generating layer comprises a binder in an amount of 10-90% by weight, and more preferably 20-75% by weight, based on the weight of the charge generating layer.

The binder is capable of dispersing or dissolving the charge transport compound (in the charge transport layer) and the charge generating compound (in the charge generating layer). Examples of suitable binders for both the charge generating layer and the charge transport layer include polystyrene-co-butadiene, modified acrylic polymers, polyvinyl acetate, styrene-alkyd resins, soya-alkyl resins, polyvinylchloride, polyvinylidene chloride, polyacrylonitrile, polycarbonates, polyacrylic acid, polyacrylates, polymethacrylates, styrene polymers, polyvinyl butyral, alkyd resins, polyamides, polyurethanes, polyesters, polysulfones, polyethers, polyketones, phenoxy resins, epoxy resins, silicone resins, polysiloxanes, poly(hydroxyether) resins, polyhydroxystyrene resins, novolak, poly(phenylglycidyl ether)-co-dicyclopentadiene, copolymers of monomers used in the above-mentioned polymers, and combinations of these materials. Polycarbonate binders are more preferred. Examples of suitable polycarbonate binders include polycarbonate A derived from bisphenol-A, polycarbonate Z derived from cyclohexylidene bisphenol, polycarbonate C derived from

methylbisphenol A, and polyestercarbonates.

The organophotoreceptor may include additional layers as well. Such layers are well known and include, for example, a barrier layer, a release layer, an adhesive layer, and a sub-layer. The release layer forms the uppermost layer of the photoconductor element with the barrier layer sandwiched between the release layer and the bottom of the photoconductive element. The adhesive layer locates and improves adhesion between the barrier layer and the release layer. The sub-layer is a charge blocking layer and locates between the conductive substrate and the photoconductive element. The sub-layer may also improve the adhesion between the conductive substrate and the photoconductive element.

Suitable barrier layers include coatings such as crosslinkable siloxanol-colloidal silica coating and hydroxylated silsesquioxane-colloidal silica coating, and organic binders such as polyvinyl alcohol, methyl vinyl ether/maleic anhydride copolymer, casein, polyvinyl pyrrolidone, polyacrylic acid, gelatin, starch, polyurethanes, polyimides, polyesters, polyamides, polyvinyl acetate, polyvinyl chloride, polyvinylidene chloride, polycarbonates, polyninyl butyral, polyvinyl acetoacetal, polyvinyl formal, polyacrylonitrile, polymethyl methacrylate, polyacrylates, polyvinyl carbazoles, copolymers of monomers used in the above-mentioned polymers, vinyl chloride/vinyl acetate/vinyl alcohol terpolymers, vinyl chloride/vinyl acetate/maleic acid terpolymers, ethylene/vinyl acetate copolymers, vinyl chloride/vinylidene chloride copolymers, cellulose polymers, and mixtures of these materials. The above organic binders optionally may contain small inorganic particles such as fumed silica, silica, titania, alumina, zirconia, or a combination of these materials, typically with a particle size in the range of 0.001-0.5 micrometers, and preferably 0.005 micrometers. A preferred barrier layer is a 1:1 mixture of methyl cellulose and methyl vinyl ether/maleic anhydride copolymer with glyoxal as a crosslinker.

The release layer topcoat may comprise any release layer composition known in the art. Preferably, the release layer is a fluorinated polymer, siloxane polymer, fluorosilicone polymer, silane, polyethylene, polypropylene, or a combination of these materials. More preferably, the release layer is formed of crosslinked silicone polymers.

Typical adhesive layers include film-forming polymers such as polyester, polyvinylbutyral, polyvinylpyrolidone, polyurethane, polymethyl methacrylate, poly(hydroxy amino ether) and the like. Preferably, the adhesive layer is formed of poly(hydroxy amino ether). If such layers are utilized, the adhesive layer preferably has a dry thickness of 0.01-5 micrometers.

Typical sub-layers include polyvinylbutyral, organosilanes, hydrolyzable silanes, epoxy resins, polyesters, polyamides, polyurethanes, silicones and the like. Preferably, the sub-layer has a dry thickness of 20-2,000 Angstroms.

The charge transport compounds and organophotoreceptors including these compounds are suitable for use in an imaging process with either dry or liquid toner development. Liquid toner development is generally preferred because it offers the advantages of providing higher resolution images and requiring lower energy for image fixing compared to dry toners. Examples of useful liquid toners are well known. The liquid toner typically includes a colorant, a resin binder, a charge director, and a carrier liquid. A preferred resin to pigment ratio is 2:1 to 10:1, more preferably 4:1 to 8:1. The colorant, resin, and charge director form toner particles.

The present invention will now be described further by way of the following examples. The following examples are for illustrative purposes and are not intended to limit the scope of the invention.

## EXAMPLE 1

### A. Synthesis

Compound (B)

To a 2-liter 3-neck round bottom flask equipped with a mechanical stirrer, a reflux condenser, and a heating mantle, 9-ethyl-3-carbazolecarboxyaldehyde (1 mole, 223.28 g, from Aldrich Chemical Company) and tetrahydrofuran (600 ml) were added. The mixture was heated to dissolve all solid contents in solution. Phenyl hydrazine (119 g, 1.1 mole, from Aldrich Chemical Company) was added, and the mixture was refluxed for 2 hours. When TLC showed that the starting materials fully disappeared to form the product, the flask was cooled to room temperature, and the solvent was removed.

The solid was filtered, washed with 20 ml of ethanol, and dried. A yellow solid was obtained (263 g, 80% yield).

To a 250-ml 3-neck round bottom flask equipped with a thermometer and a mechanical stirrer, the yellow solid (0.1 mole, 33.14 g) prepared above and DMSO (50 ml) were added. After the solid was dissolved, 1,10-dibromodecane (15 g, 0.05 mole, from Aldrich Chemical Company) was added. An aqueous solution of 50% NaOH (20 g) was added and heated to 85°C for 2 hours.

The mixture was cooled to room temperature, and then 2 L of water was added to the mixture. A light yellow solid was precipitated out, filtered, washed with water, and dried to obtain compound (B) with an yield of 6% (49 g); m.p.= 119 °C. The structure of compound (B) was identified by $^1$H-NMR spectrum.

The $^1$H-NMR spectrum of the solid showed peaks at 1.26-1.52 ppm (m ; 16H); 1.62-1.84 ppm (m ; 4H) ; 3.82-4.01 ppm (t ; 4H) ; 4.25-4.47 ppm (q , 6H) ; 6.85 - 6.96 ppm (t ; 2H); 7.09-7.25 ppm (m ; 2H) ; 7.29-7.54 ppm (m ; 12H) ; 7.70-7.79 ppm (s ; 2H) ; 7.86-7.98 ppm (dd ; 2H); 8.08-8.19 ppm (d ; 2H); 8.28-8.38 ppm (d ; 2H).

Compound (C)

Compound (C) was prepared according to the procedure of compound (B) except that 1,10-dibromodecane (0.05 mole) was replaced with 1,5-dibromodecane (0.05 mole, from Aldrich Chemical Company). The yield was 65%; m.p.= 203 °C. The structure of compound (C) was identified by $^1$H-NMR spectrum.

The $^1$H-NMR spectrum of the solid showed peaks at 1.35-1.49 ppm (t ; 6H); 1.60-1.75 ppm (m ; 2H); 1.77-1.97 ppm (m ; 4H); 3.93-4.10 ppm (t ; 4H); 4.28-4.44 ppm (q ; 4H); 6.86-6.98 ppm (t ; 2H); 7.28-7.53 ppm (m ; 14H); 7.73-7.82 ppm (s ; 2H); 7.88-7.99 ppm (dd ; 2H); 8.06-8.17 ppm (d ; 2H); 8.28-8.39 ppm (d ; 2H).

Compound (D)

Compound (D) was prepared according to the procedure of compound (B) except that 1,10-dibromodecane (0.05 mole) was replaced with 1,4-dibromodecane (0.05 mole, from Aldrich Chemical Company). The yield was 70%; m.p.= 207 °C. The structure of compound (D) was identified by $^1$H-NMR spectrum.

The $^1$H-NMR spectrum of the solid showed peaks at 1.33-1.50 ppm (t; 6H); 1.80-2.06 ppm (m ; 4H); 3.92-4.16 ppm (m ; 4H ); 4.21-4.51 ppm (q ; 4H); 6.87-7.03 ppm (t ; 2H); 7.10-7.24 ppm (m ; 2H); 7.27-7.60 ppm (m ; 14H); 7.72-7.82 ppm (s ; 2H); 7.87-7.98 ppm (dd ; 2H); 8.04-8.19 ppm (d ; 2H); 8.27- 8.39 ppm (s ; 2H).

Compound (E)

To a 3-liter 3-neck round bottom flask equipped with a reflux condenser and a mechanical stirrer, carbazole (177.78 g, 1.063 mole, from Aldrich Chemical Company), 1-bromoheptane (200 g, 1.117 mole, from Aldrich Chemical Company), and toluene (800 ml) were added. The mixture was stirred at room temperature for 30 minutes. Then the mixture was refluxed for 5 hours after 50% NaOH aqueous solution (400 g) was added. The mixture was cooled to room temperature and an organic phase appeared. The organic phase was separated, washed with water, dried over $Mg_2SO_4$, filtered, and evaporated to remove all solvent. An oil was obtained with an yield of 78% (220 g). The structure of N-heptylcarbazole was identified by $^1$H-NMR spectrum.

To a 1-liter 3-neck round bottom flask equipped with a mechanical stirrer, a dropping funnel, and a thermometer, N-heptylcarbazole (282 g, 1.062 mole, prepared in the previous step) and DMF (500 ml) were added. The flask was placed on ice bath until the temperature inside reached 5 °C, and then $POCl_3$ (109 g, 1.17 mole) was added dropwise via the dropping funnel. During the addition the temperature was controlled not to rise over 5 °C. After the addition of $POCl_3$ was completed, the flask was placed in a boiling water bath for 2 hours. Then the solution in the flask was cooled to room temperature, and 3L of water was added. The solid was filtered, washed repeatedly with water, and dried. The yield was 75%. The structure of 9-heptylcarbazole-3-carboxyaldehyde was identified by $^1$H-NMR spectrum.

To a 2-liter 3-neck round bottom flask equipped with a mechanical stirrer, a reflux condenser, and a heating mantle, 9-heptylcarbazole-3-carboxyaldehyde (1 mole, 293.45 g) and 600 ml of tetrahydrofuran were added. The mixture was heated to fully dissolve all solid contents in solution. Phenyl hydrazine (119 g, 1.1 mole, from Aldrich Chemical Company) was added, and the mixture was refluxed for 2 hours. When TLC showed that the starting material fully disappeared to form the product, the flask was cooled to room temperature and the solvent was evaporated. The solid was filtered, washed with 20 ml of ethanol, and dried. A yellow solid was obtained (249 g, 83% yield).

To a 250-ml 3-neck round bottom flask equipped with a thermometer and a mechanical stirrer, the yellow solid (0.1 mole, 38.36 g) prepared above and 50 ml of DMSO were added. After the solid was dissolved, 1,10-dibromodecane (15 g, 0.05 mole) was added. An aqueous solution of 50% NaOH (20 g) was added and heated at 85°C for 2 hours with stirring. The mixture was cooled to room temperature, and 2 L of water was added to the mixture. A light yellow solid was precipitated out, filtered, washed with water, and dried to obtain compound (E) with an yield of 55% (25%); m.p.=116 °C. The structure of compound E was identified by $^1$H-NMR spectrum.

The $^1$H-NMR spectrum of the solid showed peaks at 0.70-0.96 ppm (t ; 6H); 1.01-1.62 ppm (m ; 28H); 1.64-2.00 ppm (m ; 8H); 3.80-4.06 ppm (t ; 4H); 4.18-4.42 ppm (t ; 4H); 6.77-7.00 ppm (t ; 2H); 7.12-7.29 ppm (m ; 4H); 7.28-7.58 ppm (m ; 12H); 7.67-7.81 ppm (s ; 2H); 7.85-8.01 ppm (dd ; 2H); 8.07-8.22 ppm (d ; 2H); 8.26-8.43 ppm (s ; 2H).

Compound (F)

Compound (F) was prepared according to the procedure of compound (E) except that 1,10-dibromodecane was replaced with 1,5-dibromodecane. The yield was 45%, m.p.= 120 °C. The structure of compound (F) was identified by $^1$H-NMR spectrum.

The $^1$H-NMR spectrum of the solid showed peaks at 0.69-0.95 ppm (t ; 6H); 1.05-1.49 ppm (m ; 20H); 1.75-1.98 ppm (m ; 6H); 3.84-4.12 ppm (t; 4H); 4.16-4.40 ppm (t ; 4H); 6.86-7.00 ppm (t ; 2H); 7.15-7.29 ppm (m ; 4H); 7.37-7.51 ppm (m ; 12H); 7.71-7.83 ppm (s ; 2H); 7.88-8.00 ppm (dd ; 2H); 8.06-8.19 ppm (d ; 2H); 8.26-8.40 ppm (s, 2H).

Compound having formula (4) above

A compound having formula (4) (hereinafter, compound (4)) was prepared according to the procedure of compound (E) except that 1-bromoheptane was replaced with 1-bromo-3-propylbenzene. The yield was 60%, m.p.= 184 °C. The structure of compound (4) was identified by $^1$H-NMR spectrum.

**B. $^1$H NMR Measurements**

The $^1$H-NMR spectra were obtained by a 300 MHz Bruker NMR spectrometer (from Bruker Instruments Inc., Billerica, MA) using $CDCl_3$ solvent with 0.03% v/v tetramethylsialine (from Aldrich Chemical Company) as the internal reference. The following abbreviations were used: s for singlet; d for doublet; dd for double doublet; m for multiplet; q for quartet; and t for triplet.

**C. Thermal Transitions**

Thermal transition data for various charge transport compounds was collected using a TA Instruments Model 2929 Differential Scanning Calorimeter (New Castle, DE) equipped with a DSC cooling system (-70°C minimum temperature limit), and dry helium and nitrogen exchange gases. The calorimeter ran on a Thermal Analyst 2100 workstation with version 8.10B software. An empty aluminum pall was used as the reference.

Samples were tested both neat and as a mixture with Polycarbonate Z ("PCZ"). The neat samples were prepared by placing 4.0 to 8.0 mg of neat charge transport material into an aluminum sample pan and crimping the upper lid to produce a hermetically sealed sample for DSC testing. The results were normalized on a unit mass basis.

The Polycarbonate Z-mixed samples were prepared by filling the bottom portion of the aluminum sample pan with a 15-20% solid solution of the charge transport material in Polycarbonate Z, followed by air-drying overnight. Each air-dried sample was then placed in a convection oven at 50-55°C for 24-48 hours to fully remove the solvent, after which the upper sample lid was crimped on to produce a sealed sample for DSC testing. Each sample size was in the range of 7.0-15.0 mg. The results were normalized on a unit mass basis.

Each sample was subjected to the following protocol to evaluate its thermal transition behavior:

1. Equilibrate at 0°C (Default--Nitrogen Heat Exchange Gas);
2. Isothermal for 5 min.;
3. External Event: Nitrogen Heat Exchange Gas;
4. Ramp 10.0°C/min to a temperature 30°C above the melting point of the charge transport material;
5. External Event: Helium Heat Exchange Gas;
6. Isothermal for 5 min.;
7. Ramp 10.0°C/min to 0°C;
8. External Event: Nitrogen Heat Exchange Gas;
9. Isothermal for 5 min.;
10. Ramp 10.0°C/min to a temperature 40°C above the melting point of the charge transport material;
11. External Event: Helium Heat Exchange Gas;
12. Isothermal for 5 min.;
13. Ramp 10.0°C/min to 0°C;
14. External Event: Nitrogen Heat Exchange Gas;
15. Isothermal for 5 min;
16. Ramp 10.0°C/min to 275°C.

The first cycle (steps 1-7) is used to (a) remove the thermal history of the sample, (b) obtain the melting transition for crystalline charge transport materials, and (c) obtain a homogeneous charge transport material/Polycarbonate Z mixture in the event the charge transport material is crystallized during sample preparation. A homogeneous mixture can be obtained as long as the charge transport material (molten or casted) is miscible with the Polycarbonate-Z.

The second cycle (steps 8-13) is used to identify the glass transition temperature and the recrystallization or melting transitions of charge transport materials.

The third cycle (steps 14-16) is used to report the final thermal transitions.

The results are shown below in Table 1. All temperatures are reported in °C. The abbreviation "CTM" indicates charge transport compounds. The abbreviation "PCZ" indicates the Polycarbonate Z binder.

Table 1

| Compound | M.p (°C) | Tg without binder ( °C) | Tg with 50% PCZ ( °C) |
|---|---|---|---|
| B | 119 | 46 | 83.7 |
| C | 203 | 84.4 | 102.5 |
| D | 207 | 79.1 | NA |
| E | 116 | 29.3 | 56.9 |
| F | 120 | 44.5 | 78.3 |
| 4 | 184 | 57.4 | NA |
| NA; Not Applicable | | | |

As expected, an increase in the aliphatic chain length of $R_1$ in formula (1) from ethyl to heptyl lowers the Tg (Compare compound (B) with compound (E)). Also, an increase in the chain length of the X-Y linkage in formula (1) from pentyl to decyl also lowers the Tg (Compare compounds (B) with compound (C)). In the presence of the Polycarbonate Z binder, the Tg of compounds (D) and (4) could not be measured due to phase separation.

**D. Organphotoreceptor Preparation Methods**

**(i) Die Coating**

A charge transport solution containing 50 wt. % of a selected charge transport compound in Polycarbonate Z binder ("Lupilon Z-200" resin, from Mitsubishi Gas Chemical) was prepared by mixing either 10.0 g or 15.0 g, depending upon solubility, of the charge transport compound in 120.0 g of tetrahydrofuran with 15.0 g of Polycarbonate Z and 0.03 g of Dow Corning 510 Fluid.

The charge transport solution was then die coated onto a 3-mil (76 micrometer) thick polyethylene terephthalate (PET) film (Melinex 442 polyester film from Dupont) having a 1 ohm/square aluminum vapor coat and an additional 0.25-micrometer thick PET sub-layer overlaid with the aluminum vapor coat. The purpose of including the PET sub-layer was to improve adhesion and prevent charge diffusion into the charge transport layer. The dried charge transport layer had a nominal thickness of 8.75 micrometers. The die coating (also known as slot coating) technique described by E. Cohen and E. Gutoff, *Modern Coating and Drying Technology,* VCH Publishers, Inc. New York, 1992, pp. 117-120, was used.

A dispersion was prepared by micronising 32.6 g of oxytitanium phthalocyanine pigment (Jupiter, FI , from H. W. Sands Corp.), 32.6 g of S-Lec B Bx-5 polyvinyl butyral resin (from Sekisui Chemical Co. Ltd.), and 684.4 g of 2/1 (v/v) methyl ethyl ketone/toluene using a horizontal sand mill operating in a recirculation mode for 8 hours. This stock solution was diluted to 3.5 wt. % solid by adding 1113 g of 2:1 (v/v) methyl ethyl ketone/toluene prior to coating. The resultant dispersion was die coated onto the charge transport layer and dried to form a charge generating layer having a nominal thickness of 0.27 micrometer. This dual-layered organic photoconductor was then overcoated with a barrier layer.

Two different barrier layer solutions were used. The first barrier layer solution ("Barrier A") was prepared by mixing 86.3 g of 3% Methocel A15L V in water, 86.3 g of 3% Gantrez AN-169 polymer (from ISP Technologies) in water, 172.44 g of methanol, 0.65 g of 40% Glyoxal 40 in water, and 0.07 g Triton X-100 surfactant. The other barrier layer solution ("Barrier B") was prepared by using 217.6 g of 6% S-Lec Bx-5 polyvinyl butyral resin, 1385.7 g isopropyl alcohol, 33.5 g Nalco 1057 colloidal silica, 33.1% Z-6040 silane (Dow Coming 50/50 in isopropyl alcohol/water), and 130.17 g Gantrez AN-169 polymer according to the procedure described in U.S. Patent No. 5,733,698.

The barrier layer solution was die coated onto the dual-layered organophotoconductor and dried to form a barrier layer having a nominal thickness of 0.4 micrometer.

### ii. Lamination

Inverted dual-layered organophotoreceptors were prepared by incorporating compounds (B) through (F) and (4) as charge transport materials. A charge transport solution containing 50 wt. % of a selected charge transport compound in Polycarbonate Z binder was prepared by mixing a solution of 1.25 g of the charge transport compound in 8.0 g of tetrahydrofuran with 1.25 g of Polycarbonate Z in 2.50 g of toluene. The charge transport solution was then hand-coated with a Maier rod (# 36) onto a 3-mil (76-micrometer) thick aluminized polyethylene terephthalate film (Melinex 442 polyester film having a 1 ohm/square aluminum vapor coat, from Dupont) having a 0.3-micrometer polyester resin sub-layer (Vitel PE-2200 from Bostik, Middletown, MA) and dried to form a charge transport layer having a thickness of 9 micrometers.

A dispersion was prepared by micronising 1.35 g of oxytitanium phthalocyanine pigment (Jupiter, Fl, from H. W. Sands Corp.), 1.35 g of S-Lec B Bx-5 polyvinylbutryal resin (Sekisui Chemical Co. Ltd.), 26 g of methyl ethyl ketone, and 13 g of toluene using a horizontal sand mill operating in a recirculation mode for 8 hours. The resultant dispersion was then die coated onto a 2-mil (51-micrometer) thick polyethylene terephthalate (PET) film on which a sub-layer was not formed and dried at 80 °C for 10 minutes to form a charge generating layer having a nominal thickness of 0.27 micrometers on the PET film.

The charge transport layer and the charge generating layer were laminated together at 140°C using a Model 447 Matchprint TM Laminator (from Imation Corp., Oakdale, Minn.). After lamination, the 2-mil (51-micrometer) thick PET film was separated from the surface of the charge generation layer to form an inverted dual-layered organophotoreceptor.

### E. Solubility Testing

Solubility testing was performed for individual charge transport compounds at room temperature using tetrahydrofuran as a solvent. The solubility was determined as the percent solids of saturated solution. In general, it is desirable to maximize the solubility value.

### F. Electrostatic Testing

Electrostatic testing was performed on individual inverted dual-layered organophotoreceptor samples prepared with compounds (B) through (F) and (4) either by lamination or by die coating.

Electrostatic testing for the samples was performed and recorded on a QEA PDT-2000 instrument at ambient temperature. Charge-up was performed at 8 kV. Discharge was performed by exposing the organophotoreceptor to a 780 nm-filtered tungsten light source down a fiber optic cable. Each sample was exposed to 2 microjoules/$cm^2$ of energy for 0.05 seconds; total exposure intensity was 20 microwatts/$cm^2$. After charge-up, the acceptance voltage ($V_{acc}$) was measured in volts. This value was recorded as $V_{acc}$ after one cycle. Following this initial charge-up, a 1-second dark decay followed before the sample was discharged for 0.05 seconds with light pulses of 2 microjoules/$cm^2$ at 780 nm. Next, the residual voltage ($V_{res}$) was measured in volts. This value was recorded as $V_{res}$ after one cycle. $V_{acc}$ and $V_{res}$ were measured again after 1000 cycles. In general, it is desirable to maximize $V_{acc}$ and to minimize $V_{res}$.

Table 2

| Compound | Vacc (V) | Dark Decay (V) | Discharge (V) | Vres (V) |
|---|---|---|---|---|
| B | 575 | 64 | 398 | 58 |
| C | 512 | 109 | 393 | 50 |
| E | 596 | 38 | 89 | 468 |
| F | 560 | 151 | 322 | 91 |

The data in Table 2 indicate that these charge transport compounds are suitable for making organophotoreceptors.

<u>Compound (G)</u>

Compound (G) having the formula below was prepared:

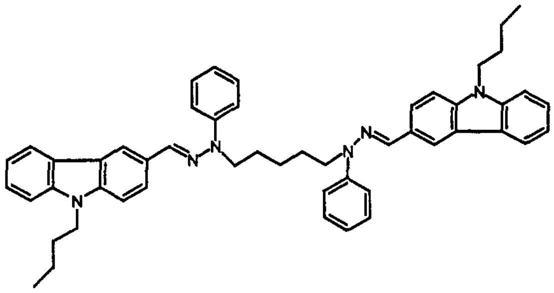

(G)

First, to a 5-liter 3-neck round bottom flask equipped with a reflux condenser, a mechanical stirrer, and a heating mantle, carbazole (579.62 g, 3.47 mole, from Aldrich Chemical Company, Milwaukee, WI), 1-bromo butane (500 g, 3.65 mole, from Aldrich Chemical Company, Milwaukee, WI), benzyltriethylammonium chloride (39.48 g, 0.17 mole, from Aldrich Chemical Company, Milwaukee, WI), and toluene (3 liter) were added. The mixture was stirred at room temperature for 30 minutes. Then 50% NaOH aqueous solution (1300 g) was added, and the mixture was refluxed for 5 hours. The mixture was cooled to room temperature, and the organic phase was separated, washed with water, dried over magnesium sulfate, filtered, and evaporated to fully remove the solvent. A liquid product was obtained. The yield was 83% (644 g). The liquid product was identified to be N-butylcarbazole by $^1$H-NMR spectrum ($CDCl_3$).

To a 3-liter 3-neck round bottom flask equipped with a mechanical stirrer, a dropping funnel, and a thermometer, N-butylcarbazole (644 g, 2.88 mole, prepared in the previous step) and DMF (1300 ml) were added. The flask was placed on ice bath until the temperature inside the flask reached 5°C, and then $POCl_3$ (498 g, 3.25 mole, from Aldrich Chemical Company) was added dropwise via the dropping funnel. During addition of $POCl_3$, the temperature was controlled not to rise over 5°C. The solution in the flask was cooled to room temperature, and excess water (3L) was added. The solid was filtered, washed repeatedly with water, and dried. The yield was 92% (668 g). The product in this step was identified to be 9-butylcarbazole-3-carboxaldyde by $^1$H-NMR spectrum ($CDCl_3$).

To a 2-liter 3-neck round bottom flask equipped with a mechanical stirrer, a reflux condenser, a heating mantle, 9-butylcarbazole (668 g, 2.66 mole, prepared in the previous step), 500 ml of toluene, and phenyl hydrazine (316.4 g , 2.93 mole, from Aldrich Chemical Company) were added. The mixture was refluxed for 2 hours. When thin layer chromatography (TLC) showed that the starting materials fully disappeared to form the product, the flask was cooled to room temperature. The solid was collected, washed with 100 ml of ethanol, and dried. The yield was 85% (773 g). The product in this step was identified to be the expected product by $^1$H-NMR spectrum ($CDCl_3$).

To a 250-ml 3-neck round bottom flask equipped with a thermometer and a mechanical stirrer, the product prepared in the previous step (34.15 g, 0.1 mole) and 50 ml of DMSO were added. After the solid was dissolved, 1,5-dibromoheptane (11.5 g, 0.05 mole, from Aldrich Chemicals Company) was added. An aqueous solution of 50% NaOH (20 g) was added, and the mixture was refluxed at 85°C for 2 hours. The mixture was cooled to room temperature, and 2L of water was added and left at room temperature. The resultant gully solid was washed repeatedly with water and dried. A crude product (20 g) was obtained. The crude product was recrystallized 4 times with activated charcoal, silica, and ethyl acetate, and dried in a vacuum oven at 80°C for six hours to obtain a light yellow solid compound (G). The melting point of compound (G) was found to be 182-183°C. The structure of compound (G) was identified by $^1$H-NMR spectrum.

The $^1$H- NMR spectrum of the final product in $CDCl_3$ showed peaks at 0.78-1.07 ppm (t ; 6H) ; 1.31-1.49 ppm (m; 4H) ; 1.48-1.59 ppm (m ; 2H) ; 1.59-1.74 ppm (m ; 2H) ; 1.75-1.99 ppm (m , 6H) ; 3.91-4.08 ppm (t ; 4H) ; 4.22-4.37 ppm (t ; 4H) ; 6.79-7.03 ppm (t ; 2H) ; 7.14-7.29 ppm (t ; 2H) ; 7.29-7.54 ppm (m ; 14H) ; 7.71-7.85 ppm (s ; 2H) ; 7.87-8.02 ppm (dd ; 2H) ; 8.05-8.21 ppm (d ; 2H) ; 8.25-8.43 ppm (d ; 2H).

Compound (H)

Compound (H) having the following formula was prepared according to the procedure of compound (G) except that 1,5-dibromoheptane (0.05 mole) was replaced with 1,6-dibromohexane (0.05 mole, from Aldrich Chemical Company):

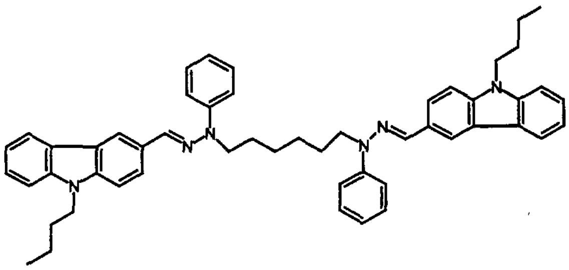

(H)

The melting point of compound (H) was found to be 186-187°C. The structure of compound (H) was identified by $^1$H-NMR spectrum.

The $^1$H-NMR spectrum of the final product in $CDCl_3$ showed peaks at 0.84-1.05 ppm (t ; 6H) ; 1.32-1.48 ppm (m ; 4H) ; 1.48-1.69 ppm (m ; 4H) ; 1.73-1.99 ppm (m ; 8H) ; 3.90-4.08 ppm (t ; 4H) ; 4.21- 4.40 ppm (t ; 4H) ; 6.84-6.97 ppm (t ; 2H) ; 7.15-7.25 ppm (t ; 2H) ; 7.29-7.56 ppm (m ; 14H) ; 7.71-7.84 ppm (s ; 2H) ; 7.84-7.99 ppm (dd ; 2H) ; 8.06-8.22 ppm (d ; 2H) ; 8.26-8.43 ppm (d ; 2H).

Compound (I)

Compound (I) having the following formula was prepared according to the procedure of compound (G) except that 1,5-dibromoheptane (0.05 mole) was replaced with 1,8-dibromooctane (0.05 mole, from Aldrich Chemical Company):

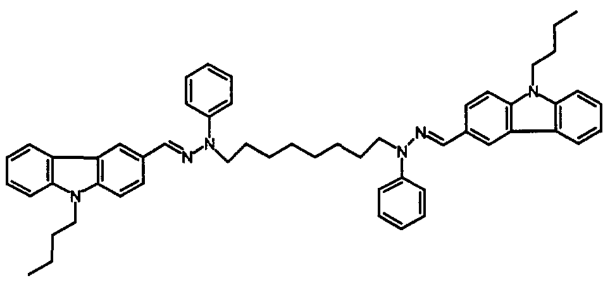

(I)

The structure of compound (I) was identified by $^1$H-NMR spectrum. The $^1$H-NMR spectrum of the final product in $CDCl_3$ showed peaks at 0.80-1.09 ppm (t ; 6H) ; 1.19-1.98 ppm (m ; 20 H) ; 3.85-4.07 ppm (t ; 4H) ; 4.17-4.40 ppm (t ; 4H) ; 6.83-6.97 ppm (t ; 2H) ; 7.13-7.56 ppm (m ; 16 H) ; 7.69-7.83 ppm (s ; 2H) ; 7.85-7.99 ppm (dd ; 2H) ; 8.05-8.20 ppm (d ; 2H) ; 8.25-8.41 ppm (d ; 2H).

**G. Ionization Potential Protocol**

Samples for ionization potential (Ip) measurements were prepared by dissolving compounds (B), (C), (E), (F), (G), and (H) and Comparative Example A separately in tetrahydrofuran. Each solution was hand-coated on an aluminized polyester substrate that was precision coated with a methylcellulose-based adhesive sub-layer to form a charge transport material (CTM) layer. The role of this sub-layer was to improve adhesion of the CTM layer, to retard crystallization of CTM, and to eliminate the electron photoemission from the Al layer through possible CTM layer defects. No photoemission was detected from the Al layer through the sub-layer at illumination with up to 6.4 eV quantum energy light. In addition, the adhesive sub-layer was conductive enough to avoid charge accumulation on it during measurement. The thickness of both the sub-layer and CTM layer was about 0.4 µm. No binder material was used in the preparation of the samples for Ip measurements. In most cases, the thin CTM layers were in a meta-stable amorphous phase, delaying crystallization for several hours, so that the measurement of the samples was possible. In some cases, however, crystallization began immediately after coating, as was observed with compound (H). The CTM layer formed with compound (H) was amorphous but crystal inclusions were observed at the time of the Ip measurement.

The ionization potential was measured by the electron photoemission in an air method similar to that described in "Ionization Potential of Organic Pigment Film by Atmospheric Photoelectron Emission Analysis", Electrophotography,

28, No. 4, p. 364. (1989) by E. Miyamoto, Y. Yamaguchi, and M. Yokoyama, which is hereby incorporated by reference. The samples were illuminated with monochromatic light from a quartz monochromator with a deuterium lamp source. The power of the incident light beam was 2-5$\times 10^{-8}$ W. The negative voltage of -300 V was supplied to the sample substrate. The counter-electrode with the 4.5$\times$15 $mm^2$ slit for illumination was placed at 8 mm distance from the sample surface. The counter-electrode was connected to the input of the BK2-16 type electrometer, working in the open input regime, for the photocurrent measurement. A photocurrent of $10^{-15}$-$10^{-12}$ amp was flowed in the circuit under illumination. The photocurrent, I, was strongly dependent on the incident light photon energy hν. The $I^{0.5}$=f(hν) dependence was plotted. Usually the dependence of the square root of photocurrent on incident light quantum energy is well described by linear relationship near the threshold [see references "Ionization Potential of Organic Pigment Film by Atmospheric Photoelectron Emission Analysis", Electrophotography, 28, No. 4, p. 364. (1989) by E. Miyamoto, Y. Yamaguchi, and M. Yokoyama; and "Photoemission in Solids", Topics in Applied Physics, 26, 1-103. (1978) by M. Cordona and L. Ley]. The linear part of this dependence was extrapolated to the hν axis, and Ip value was determined as the photon energy at the interception point. The ionization potential measurement had an error of ±0.03 eV. The results are shown in Table 3.

**H. Hole Mobility**

Samples for charge carrier mobility measurements were prepared by dissolving compound (B), (C), (E), (F), and (G) and Comparative Example A separately in tetrahydrofuran with a binder to form 10% solid solutions. The binder was Polycarbonate Z 200 (from Mitsubishi Engineering Plastics, White Plains, NY). The sample to binder ratio was 4:6 or 5:5. Each solution was coated on an aluminized polyester substrate to form a charge transport material (CTM) layer. The thickness of the CTM layer varied in the range of 5-10 μm. The mobility for the sample prepared with compound (H) could not be measured because it had limited solubility.

The hole drift mobility was measured by a time of flight technique as described in "The Discharge Kinetics of Negatively Charged Se Electrophotographic Layers," Lithuanian Journal of Physics, 6, p. 569-576 (1966) by E. Montrimas, V. Gaidelis, and A. Paž era, which is hereby incorporated by reference. Positive corona charging created electric field inside the CTM layer. Charge carriers were generated at the layer surface by illumination with pulses of nitrogen laser (pulse duration was 2 ns, wavelength 337 nm). The layer surface potential decreased, as a result of pulse illumination, to 1-5 % of the initial potential before illumination. The capacitance probe that was connected to the wide frequency band electrometer measured the speed of the surface potential dU/dt. The transit time $t_t$ was determined by the change (kink) in the curve of the dU/dt transient in linear or double logarithmic scale. The drift mobility was calculated by the formula $\mu = d^2/U_0 \cdot t_t$, where d is the layer thickness and $U_0$ is the surface potential at the moment of illumination.

Mobility values at electric field strength, E, of 6.4$\times 10^5$ V/cm are given in Table 3. The mobility field dependencies may be approximated by the function

$$\mu \sim e^{\alpha\sqrt{E}}$$

where α is parameter characterizing mobility field dependence. The value of the parameter α is also given in Table 3.

Table 3

| Compound | Solubility | Mobility μ, $cm^2$/V.s at 6.4$\times 10^5$ V/cm | Mobility Relative to HCTM1 | α | Ip, eV |
|---|---|---|---|---|---|
| B | Soluble w/ heat | 40:60 | | | 5.44 |
| C | Soluble w/ heat | 1.1$\times 10^{-5}$ at 40:60 with PC | 1.22 | | 5.34 |
| E | Soluble w/o heat | 1.7$\times 10^{-5}$ at 40:60 with PC | 1.88 | ~0.006 | 5.38 |
| F | Soluble w/o heat | 6.5$\times 10^{-6}$ at 40:60 with PC | 0.72 | 0.0053 | 5.23 |
| G | Soluble w/o | 3.6$\times 10^{-6}$ at 40:60, | 0.40 | ~0.007 | 5.4 |

Table 3 (continued)

| Compound | Solubility | Mobility μ, $cm^2$/V.s at $6.4\times10^5$ V/cm | Mobility Relative to HCTM1 | α | Ip, eV |
|---|---|---|---|---|---|
| | heat | $5.6\times10^{-6}$ at 50:50 with PC | 0.62 | 0.009 | |
| H | Insoluble | - | - | - | 5.35, layer with crystal inclusions |
| Comparative Example A* | - | $5.2\times10^{-6}$ at 40:60 with PC | 0.58 | ~0.006 | |
| | | $9\times10^{-6}$ at 50:50, | 1.00 | 0.0055 | 5.23 |

* Comparative Example A was Compound 2 in U.S. Patent No. 6,140,004, which is hereby incorporated by reference.

**I. Extended Electrostatic Cycling**

Extended electrostatic cycling was performed using an in-house designed and developed test bed that tests up to 3 samples strips that were wrapped around a drum. The three coated sample strips, each measuring 50 cm long by 8.8 cm wide, were fastened side-by-side and completely around an aluminum drum (50.3-cm circumference). At least one of the strips was a control sample (compound (M)) that was precision web coated and used as an internal reference point. In this electrostatic cycling tester, the drum rotated at a rate of 8.13 cm /min (3.2ips) and the location of each station in the tester (distance and elapsed time per cycle) is given in Table 4:

Table 4.

| Electrostatic test stations around the sample sheet wrapped drum. | | | |
|---|---|---|---|
| Station | Degrees | Total Distance, cm | Total Time, sec |
| Front erase bar edge | 0° | Initial, 0 cm | Initial, 0 s |
| Erase Bar | 0-7.2° | 0-1.0 | 0 - 0.12 |
| Scorotron | 113.1 - 135.3° | 15.8 - 18.9 | 1.94 - 2.33 |
| Laser Strike | 161.0° | 22.5 | 2.77 |
| Probe #1 | 181.1° | 25.3 | 3.11 |
| Probe #2 | 251.2° | 35.1 | 4.32 |
| Erase bar | 360° | 50.3 | 6.19 |

The first electrostatic probe (Trek 344 electrostatic meter, from Trek Inc., Medina, NY) was located 0.34 s after the laser strike station and 0.78 s after the scorotron. Also, the second probe (Trek 344 electrostatic meter) was located 1.21 s from the first probe and 1.99 s from the scorotron. All measurements were performed at ambient temperature and relative humidity.

Electrostatic measurements were obtained as a compilation of several tests. The first three diagnostic tests (prodstart, VlogE initial, dark decay initial) were designed to evaluate the electrostatic cycling of a new, fresh sample and the last three, identical diagnostic tests (prodend, VlogE final, dark decay final) were run after cycling of the sample (longrun).

1) PRODSTART: The erase bar was turned on during this diagnostic test and the sample recharged at the beginning of each cycle (except where indicated as scorotron off). The test sequence was as follows. The sample was completely charged for three complete drum revolutions (laser off); discharged with the laser at 780nm & 600dpi on the forth cycle; completely charged for the next three cycles (laser off); discharged with only the erase lamp at 720nm on the eighth cycle (corona and laser off); and, finally, completely charged for the last three cycles (laser off).
2) VLOGE: This test measures the photoinduced discharge of the photoconductor to various laser intensity levels by monitoring the discharge voltage of the belt as a function of the laser power (exposure duration of 50 ns). The complete sample was charged and discharged at incremental laser power levels per each drum revolution. A semi-logarithmic plot was generated (voltage versus log E) to identify the sample's sensitivity and operational power settings.

3) DARK DECAY: This test measures the loss of charge acceptance with time without laser or erase illumination and can be used as an indicator of (i) the injection of residual holes from the charge generation layer to the charge transport layer, (ii) the thermal liberation of trapped charges, and (iii) the injection of charge from the surface or aluminum ground plane. After the belt has been completely charged, it was stopped, and the probes measured the surface voltage over a period of 90 seconds. The decay in the initial voltage was plotted versus time.

4) LONGRUN: The belt was electrostatically cycled for 100 drum revolutions according to the following sequence per each belt-drum revolution. The belt was charged by the corona, the laser was cycled on and off (80-100° sections) to discharge a portion of the belt and, finally, the erase lamp discharged the whole belt in preparation for the next cycle. The laser was cycled so that the first section of the belt was never exposed, the second section was always exposed, the third section was never exposed, and the final section was always exposed. This pattern was repeated for a total of 100 drum revolutions and the data for every $5^{th}$ cycle was recorded.

5) After the 100th cycle (long run test), the PRODSTART (now called PRODEND), VLOGE, DARK DECAY diagnostic tests were run again.

Table 5 shows the results of the prodstart and prodend diagnostic tests. The values for the charge acceptance voltage ($V_{acc}$, probe #1 average voltage obtained from the third cycle), discharge voltage ($V_{dis}$, probe #1 average voltage obtained from the fourth cycle), functional dark decay voltage ($V_{dd}$, average voltage difference between probes 1 & 2 obtained from the third cycle), and the residual voltage ($V_{res}$, average voltage obtained from the eighth cycle) are reported for the initial and final (post $100^{th}$ cycle) cycles.

Table 5.

| Electrostatic cycling of knife-coated inverted dual layer constructions. | | | | | | | | |
|---|---|---|---|---|---|---|---|---|
| Compound | $V_{acc}$, intial | $V_{acc}$, final | $V_{dd}$, intial | $V_{dd}$, final | $V_{dis}$, intial | $V_{dis}$, final | $V_{res}$, intial | $V_{res}$, final |
| B | 526 | 548 | 39 | 43 | 100 | 113 | 43 | 57 |
| C | 495 | 509 | 44 | 44 | 67 | 73 | 22 | 31 |
| E | 639 | 686 | 54 | 46 | 545 | 612 | 434 | 488 |
| F | 546 | 568 | 40 | 38 | 104 | 127 | 42 | 67 |
| H | 513 | 550 | 53 | 49 | 218 | 257 | 119 | 158 |
| I | 539 | 576 | 38 | 34 | 161 | 203 | 77 | 123 |
| Comparative Example A* | 568 | 577 | 32 | 36 | 57 | 62 | 16 | 23 |
| Comparative Example A* | 500 | 525 | 17 | 26 | 73 | 81 | 22 | 30 |

* Comparative Example A was Compound 2 in U.S. Patent No. 6,140,004.

## EXAMPLE 2

### A. Synthesis

Compound (M) with m=5 was prepared as follows. To a 2-liter 3-neck round bottom flask equipped with a mechanical stirrer and a reflux condenser, 112.0 g (0.41 mole) of 4-(diphenylamino)benzaldehyde (from Aldrich, Milwaukee, WI and 400 ml of THF were added. 48.77 g (0.45 mole) of Phenylhydrazine (from Aldrich, Milwaukee, WI) was added to the mixture. The solution was refluxed for 2 hours. After cooled to room temperature, the solvent was evaporated from the solution till the volume of the solution was 50 ml. Then a solid was precipitated by the addition of ethanol (50 ml), collected by filtration, washed with ethanol (50 ml), and dried in a vacuum oven at 60° C for 6 hours. The yield was 98% (146 g). The structure of the solid product was identified to be the expected product by $^1$H-NMR spectrum ($CDCl_3$) and 1R spectrum.

The $^1$H-NMR spectrum of the solid showed peaks at 3.8 ppm (N-H) (s ; 1H) and 7.19-7.55 ppm (m ; 20 H). The IR spectrum of the solid showed peaks at 1688 $cm^{-1}$ (C=O) and 3293 $cm^{-1}$ (N-H).

To a 250-ml 3-neck round bottom flask equipped with a mechanical stirrer and a thermometer, DMSO (100 ml) and the solid product prepared in the previous step (18.17 g, 0.05 mole) were added. The solution was heated at 30°C until the solid product dissolved into solution. 1,5-Dibromopentane (5.75 g, 0.025 mole) (from Aldrich, Milwaukee,

WI) was added to the solution and then 25% NaOH aqueous solution (20 g) was added. This solution was heated at 70-80°C for 4 hours. After cooled to room temperature, a gummy material was observed in the bottom of the 3-neck round bottom flask. The liquid above the gummy material was removed by decantation. The remaining gummy material was washed repeatedly with water to form a solid. The solid was recrystallized first with activated charcoal and toluene/ethanol (50:50 by volume). The material was recrystallized for the second and third time with activated charcoal, ethanol, and silica gel (added only in the third recrystallization). The product was dried in a vacuum oven at 50°C for 6 hours. The yield was 40% (7.90 g). The melting point of the product was found to be 77°C. The structure of the product was identified to be the expected product by $^1$H-NMR spectrum ($CDCl_3$) and IR spectrum.

The $^1$H-NMR spectrum of the solid showed peaks at 1.51-1.59 ppm (m ; 2H); 1.73-1.81 ppm (t ; 4H); 3.86-3.99 ppm (t ; 4H); and 6.95-7.64 ppm (m ; 40H). The IR spectrum showed peaks at 2847 $cm^{-1}$, 2910 $cm^{-1}$, and 3031 $cm^{-1}$.

### B. $^1$H NMR Measurement

The $^1$H-NMR spectra were obtained by a 300 MHz Bruker NMR spectrometer (from Bruker Instruments Inc., Billerica, MA) using $CDCl_3$ solvent with 0.03% v/v tetramethylsialine (from Aldrich Chemical Company) as an internal reference. The following abbreviations were used: s for singlet; d for doublet; dd for double doublet; m for multiplet; q for quartet; and t for triplet.

### C. IR Spectrum Measurement

The IR sample was obtained by placing a $CH_2Cl_2$ solution of the sample on an IR card (3M disposable substrate type 61 polyethylene, from 3M, St. Paul, MN). The IR spectrum was obtained by a Perkin Elmer 16 PC FT-IR Spectrometer (from Perkin Elmer, Norwalk, Connecticut).

### D. Melting Point Measurement

Melting Point of compound (M) with m=5 was collected using a TA Instrument Model 2929 Differential Scanning Calorimeter (New Castle, DE) equipped with a DSC cooling system (!70°C minimum temperature limit), and dry helium and nitrogen exchange gases. The calorimeter ran on a Thermal Analyst 2100 workstation with version 8.10B software. An empty aluminum pall was used as the reference.

The samples were prepared by placing 4.0 to 8.0 mg of compound M with m=5 into an aluminum sample pan and crimping the upper lid to produce a sealed sample for DSC testing. The results were normalized on a unit mass basis.

1. Each sample was subjected to the following protocol to evaluate its thermal transition behavior:

   1. Equilibrate at 0°C (Default--Nitrogen Heat Exchange Gas);
   2. Isothermal for 5 min;
   3. External Event: Nitrogen Heat Exchange Gas;
   4. Ramp 10.0°C/min to a temperature 200°C;

### E. Organophotoreceptor Preparation Method

Inverted dual-layered organophotoreceptors were prepared incorporating compound (M) prepared above and Comparative Example A (formula (3) of U.S. Patent No. 6,066,426) by the method disclosed in U.S. Patent No. 6,066,426. A charge transport solution containing 50 wt. % of compound M with m=5 in Polycarbonate Z binder was prepared by mixing a solution of 1.25 g of compound M with m=5 in 8.0 g of tetrahydrofuran with 1.25 g of Polycarbonate Z in 2.50 g of toluene. The charge transport solution was then hand-coated with a Maier rod (# 40) onto a 76-micrometer (3-mil) thick aluminized polyethylene terephthalate film (Melinex 442 polyester film from Dupont having a 1 ohm/square aluminum vapor coat) having a 0.3-micron thick polyester resin sub-layer (Vitel PE-2200 from Bostik, Middletown, MA) and dried to form a charge transport layer having a thickness of 9 micrometers.

A dispersion was prepared by micronising 1.35 g of oxytitanium phthalocyanine pigment (H.W. Sands Corp., Jupiter, Fl), 1.35 g of S-Lec B Bx-5 polyvinylbutryal resin (Sekisui Chemical Co. Ltd.), 26 g of methyl ethyl ketone, and 13 g of toluene using a horizontal sand mill operating in a recirculation mode for 8 hours. The resultant dispersion was then die coated onto a 2-mil (51-micrometer) thick polyethylene terephthalate (PET) film on which a sub-layer was not formed and dried at 80°C for 10 minutes to form a charge generating layer having a nominal thickness of 0.27 micrometer on the PET film.

The charge transport layer and the charge generating layer were laminated together at 140°C using a Model

447 Matchprint TM Laminator (Imation Corp., Oakdale, Minn.). After lamination, the 2-mil (51-micrometer) thick PET film was separated from the surface of the charge generation layer to form an inverted dual layer organophotoreceptor.

**F. Electrostatic Testing**

Electrostatic testing was performed on inverted dual-layered organophotoreceptor samples prepared with compound (M) with m=5 and a control sample either by lamination or by die coating.

Electrostatic testing was performed and recorded on a QEA PDT-2000 instrument at ambient temperature. Charge-up was performed at 8 kV. Discharge was performed by exposing the photoreceptor to a 780 nm-filtered tungsten light source down a fiber optic cable. Each sample was exposed to 2 microjoules/$cm^2$ of energy for 0.05 seconds; total exposure intensity was 20 microwatts/$cm^2$. After charge-up, the acceptance voltage ($V_{acc}$) was measured in volts. This value was recorded as $V_{acc}$ after one cycle.

Following this initial charge-up, a 1-second dark decay followed before the sample was discharged for 0.05 seconds with light pulses of 2 microjules/$cm^2$ at 780 nm, one second after which the decrease in voltage (Contrast) was measured in volts. Then the charge on the sample was further reduced by an eraser lamp. The final residual voltage ($V_{res}$) on the sample was measured in volts. $V_{acc}$ and $V_{res}$ were measured again after 1000 cycles. In general, it is desirable to maximize $V_{acc}$ and to minimize $V_{res}$.

Table 5

| Sample | $V_{acc}$(V) | Dark Decay (V) | $V_{res}$ (V) | Contrast (V) |
|---|---|---|---|---|
| Compound M (m=5) | 363 | 142 | 18 | 195 |
| Comparative Example A | 377 | 135 | 16 | 211 |

The data in Table 3 indicate that compound M with m=5 is suitable for making organophotoreceptors.

Compound (O)

The first step was the preparation of a 3-formyl-9-ethylcarbazole by Vilsmeier reaction. 9-Ethylcarbazole (Aldrich Chemical Company, Milwaukee, WI) was dissolved in dimethylformamide (DMF), and the solution was then cooled. Phosphorus oxychloride ($POCl_3$) (10-15% excess) was added dropwise via a dropping funnel to the cooled DMF solution. 3-Formyl-9-ethylcarbazole was isolated and purified.

To a 2-liter 3-neck round bottom flask equipped with a mechanical stirrer and a reflux condenser, and a heating mantle, 3-formyl-9-ethylcarbazole (1 mole, 223 g) and tetrahydrofuran (600 ml) were added and heated to fully dissolve all solid contents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated. 3-Formyl-9-ethylcarbazole hydrazone was isolated and purified.

To a 2-liter 3-neck round bottom flask equipped with a mechanical stirrer, a reflux condenser, and a heating mantle, 4-(diphenylamino)benzaldehyde (1 mole, 273 g, from Fluke, Milwaukee, WI) and tetrahydrofuran (600 ml) were added and heated to fully dissolve all solid contents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated. 4-(Diphenylamino)benzaldehyde hydrazone was isolated and purified.

To a 2-liter 3-neck round bottom flask equipped with a mechanical stirrer, a reflux condenser, and a heating mantle, 4-quinolinecarboxaldehyde (1 mole, 157.17 g, from Aldrich Chemical Company, Milwaukee, WI) and tetrahydrofuran (600 ml) were added and heated to fully dissolve all solid convents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated. 4-Quinolinecarboxaldehyde hydrazone was isolated and purified.

The last step was the reaction of 3-formyl-9-ethylcarbazole hydrazone, 4-(diphenylamino)benzaldehyde hydrazone, and 4-quinolinecarboxaldehyde hydrazone obtained above with a 1,2,3-tribromopropane to obtain compound (O). 3-Formyl-9-ethylcarbazole hydrazone was dissolved in DMSO. After the addition of 25% aqueous solution of NaOH, 1,2,3-tribromopropane was added to the solution. The molar ratio of 3-formyl-9-ethylcarbazole hydrazone to 1,2,3-tribromopropane was 1:1. This solution was stirred at 70°C for approximately 1 hour. 4-(Diphenylamino)-benzaldehyde hydrazone was added to the solution. The molar ratio of 4-(diphenylamino)benzaldehyde hydrazone to 1,2,3-tribromopropane was 1:1. After the addition of 4-(diphenylamino)-benzaldehyde hydrazone, the solution was heated at

70°C for one hour. 4-quinolinecarboxaldehyde hydrazone was added to the solution. The molar ratio of 4-quinolinecarboxaldehyde hydrazone to 1,2,3-tribromopropane was 1:1. After the addition of 4-quinolinecarboxaldehyde hydrazone, the solution was heated at 70 °C for one hour. The product from this reaction was isolated and purified.

Compound (P)

The first step was the preparation of 3-formyl-9-ethylcarbazole by Vilsmeier reaction. 9-Ethylcarbazole (from Aldrich Chemical Company, Milwaukee, WI) was dissolved in dimethylformamide (DMF), and the solution was then cooled. Phosphorus oxychloride ($POCl_3$) (10-15% excess) was added dropwise via a dropping funnel to the cooled DMF solution. 3-Formyl-9-ethylcarbazole was isolated and purified.

To a 2-liter 3-neck round bottom flask equipped with a mechanical stirrer, a reflux condenser, and a heating mantle, 3-formyl-9-ethylcarbazole (1 mole, 223 g) and tetrahydrofuran (600 ml) were added and heated to fully dissolve all solid contents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated. 3-Formyl-9-ethylcarbazole hydrazone was isolated and purified.

To a 2-liter 3-neck round bottom flask equipped with a mechanical stirrer, a reflux condenser, and a heating mantle, 4-(diphenylamino)benzaldehyde (1 mole, 273 g, from Fluke, Milwaukee, WI) and tetrahydrofuran (600 ml) was added and heated to fully dissolve all solid contents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated.

4-(Diphenylamino)benzaldehyde hydrazone was isolated and purified.

To a 2-liter 3-neck round bottom flask equipped with a mechanical stirrer, a reflux condenser, and a heating mantle, 9-formyl-julolidine (1 mole, 201 g, from Aldrich Chemical Company, Milwaukee, WI) and tetrahydrofuran (600 ml) were added and heated to fully dissolve all solid contents into solution. Phenyl hydrazine (119 g, 1.1 mole, from Aldrich Chemical Company, Milwaukee, WI) was added, and the mixture was refluxed for 2 hours. When TLC showed that the starting materials fully disappeared to form the product, the flask was cooled to room temperature, and the solvent was evaporated. 9-Formyl-julolidine hydrazone was isolated and purified.

The last step was the reaction of 3-formyl-9-ethylcarbazole hydrazone, 4-(diphenylamino)benzaldehyde hydrazone, and 9-formyl-julolidine hydrazone obtained above with 1,2,3-tribromopropane to obtain compound (P). 3-Formyl-9-ethylcarbazole hydrazone was dissolved in DMSO. After the addition of 25% aqueous solution of NaOH, 1,2,3-tribromopropane was added to the solution. The molar ratio of 3-formyl-9-ethylcarbazole hydrazone to 1,2,3-tribromopropane was 1:1. This solution was stirred at 70°C for approximately 1 hour. 4-(Diphenylamino)-benzaldehyde hydrazone was added to the solution. The molar ratio of 4-(diphenylamino)benzaldehyde hydrazone to 1,2,3-tribromopropane was 1:1. After the addition of 4-(diphenylamino)benzaldehyde hydrazone, the solution was heated at 70 °C for one hour. 9-Formyl-julolidine hydrazone was added to the solution. The molar ratio of 9-formyl-julolidine hydrazone to 1,2,3-tribromopropane was 1:1. After the addition of 9-formyl-julolidine hydrazone, the solution was heated at 70°C for one hour. The product from this reaction was isolated and purified.

While this invention has been particularly shown and described with reference to preferred embodiments thereof, it will be understood by those skilled in the art that various changes in form and details may be made therein without departing from the spirit and scope of the invention as defined by the appended claims.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

**Claims**

1. A charge transport compound having formula (1):

$$(R\text{-}Q)_n\text{-}Y \qquad (1)$$

where R is selected from the group consisting of julolidine ring groups, carbazole ring groups, and triarylmethane ring groups, Q is an aromatic hydrazone linking group, Y is a bridging group or chemical bond between R-Q-groups, and n is an integer from 2 to 6.

2. The charge transport compound of claim 1, wherein Q in formula (1) comprises an aromatic linking group having the following formula:

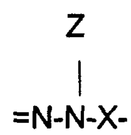

where Z is an aromatic, preferably $C_6$ to $C_{20}$ aryl, group and X is a linking group.

3. The charge transport compound of claim 2, wherein Z is a phenyl group.

4. The charge transport compound of claim 2 or 3, wherein X is $-(CH_2)_m-$, where m is an integer from 0 to 20.

5. The charge transport compound of claim 2 or 4, wherein X is a substituted or unsubstituted alkylene group, preferably a methylene group.

6. The charge transport compound of claim 5, wherein the alkylene group comprises at least one methylene group substituted with an oxygen atom, a carbonyl group, urethane, urea, an ester group, a $-NR_6$ group, a $CHR_7$ group, or a $CR_8R_9$ group, where $R_6$, $R_7$, $R_8$, and $R_9$ are, independently, H, a $C_6$-$C_{20}$ alkyl group, or a $C_6$-$C_{20}$ aryl group.

7. The charge transport compound of any preceding claim, wherein Y in formula (1) is a chemical bond, carbon atom, nitrogen atom, oxygen atom, sulfur atom, a branched or linear $-(CH_2)_p-$ group where p is an integer from 0 to 10, a $C_6$-$C_{20}$ aryl group, a $C_5$-$C_{20}$ cycloalkyl group, a cyclosiloxyl group, a heterocyclic group, or a $CR_{10}$ group where $R_{10}$ is hydrogen atom, a $C_1$-$C_{20}$ alkyl group, or a $C_6$-$C_{20}$ aryl group.

8. The charge transport compound of any preceding claim, wherein the charge transport compound is a compound having formula (2):

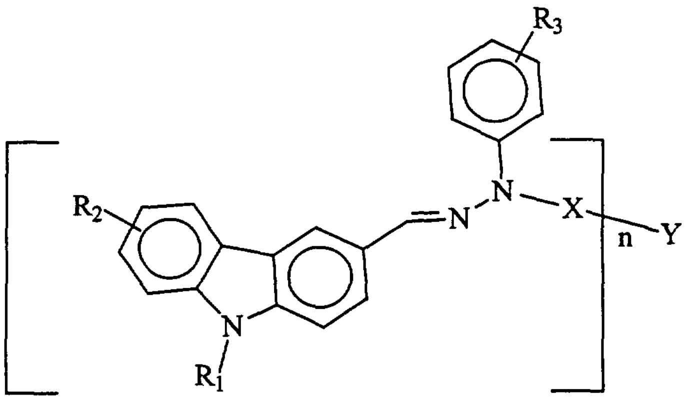

...(2)

where

n is an integer from 2 to 6;
$R_1$ is hydrogen, a branched or linear $C_1$-$C_{20}$ alkyl group, a branched or linear unsaturated $C_2$-$C_{20}$ hydrocarbon group, an ether group, or a $C_6$-$C_{20}$ aryl group;
$R_2$ is hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a $C_5$-$C_{20}$ cycloalkyl group, an aryl group, or a $-NR_4R_5$ group, where $R_4$ and $R_5$ are, independently, hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, a $C_6$-$C_{20}$ aryl group, or $R_4$ and $R_5$ form a ring in combination with nitrogen atom;
$R_3$ is hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a $C_5$-$C_{20}$ cycloalkyl group, or a $C_6$-$C_{20}$ aryl group;
X is a linking group having branched or linear $-(CH_2)_m-$, where m is an integer from 0 to 20, and at least one methylene groups is optionally substituted with oxygen atom, a carbonyl group, a urethane group, a urea group, an ester group, a $-NR_6$ group, a $CHR_7$ group, or a $CR_8R_9$ group where $R_6$, $R_7$, $R_8$, and $R_9$ are, independently, H, a $C_1$-$C_{20}$ alkyl group, or a $C_6$-$C_{20}$ aryl group; and
Y is a chemical bond, carbon atom, nitrogen atom, oxygen atom, sulfur atom, a branched or linear $-(CH_2)_p-$ group where p is an integer from 0 to 10, a $C_5$-$C_{20}$ aryl group, a $C_5$-$C_{20}$ cycloalkyl group, a cyclosiloxyl group, a heterocyclic group, or a $CR_{10}$ group where $R_{10}$ is hydrogen atom, a $C_1$-$C_{20}$ alkyl group, or a $C_6$-$C_{20}$ aryl group.

**9.** The charge transport compound of claim 8, wherein the compound having formula (2) is selected from compounds (A) through (F) having one of the following formulae:

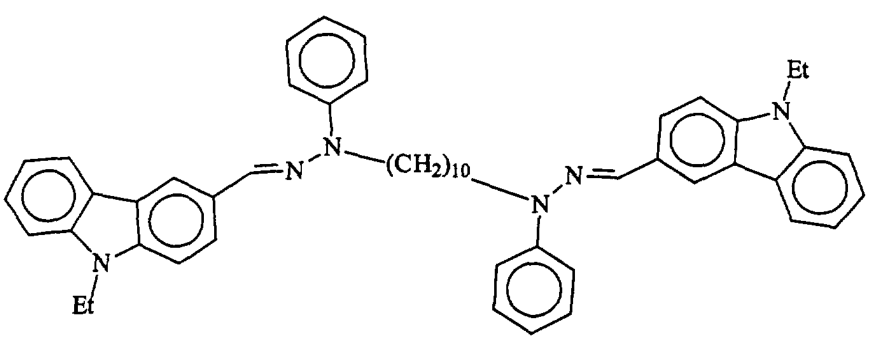

(A)

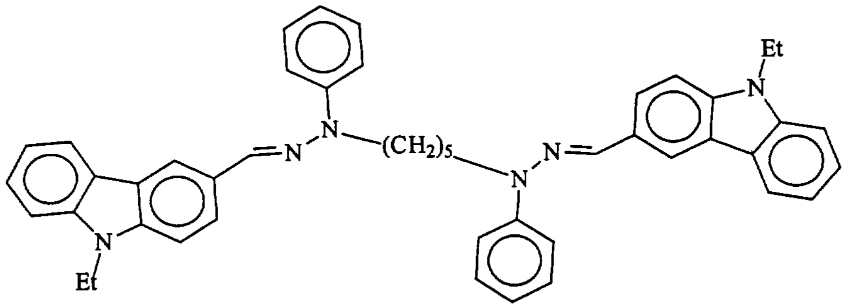

(B)

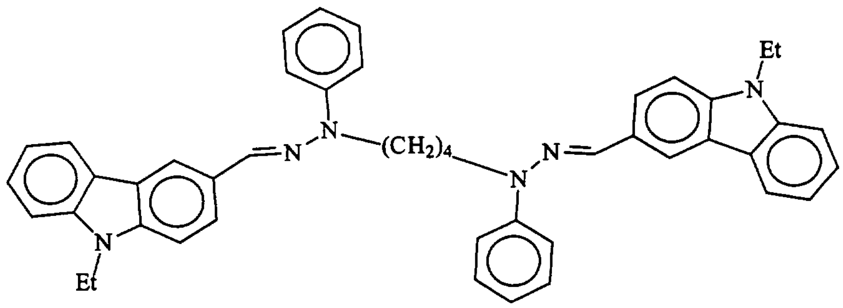

(C)

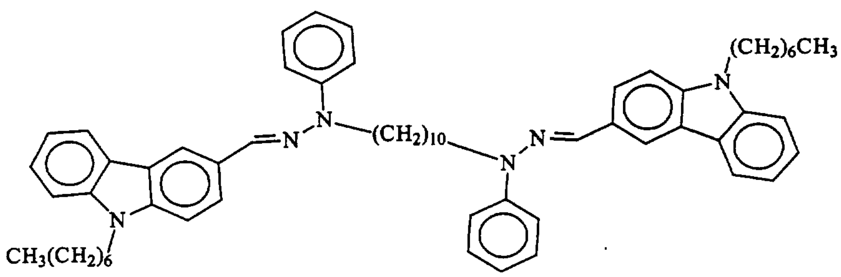

(D)

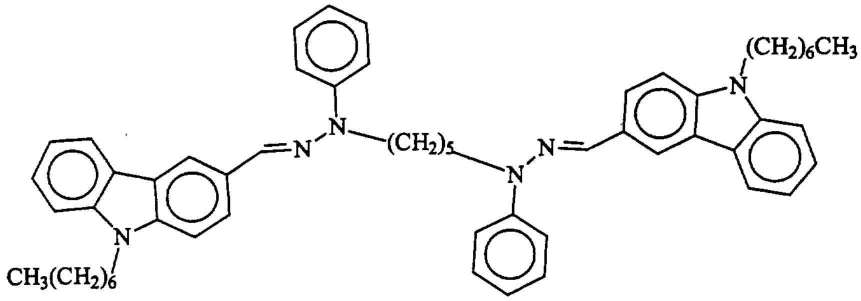

(E)

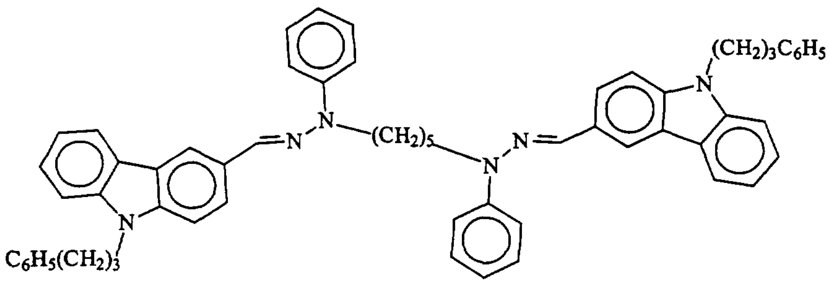

(F)

**10.** The charge transport compound of any of claims 1 to 7, wherein the charge transport compound is a compound having formula (3):

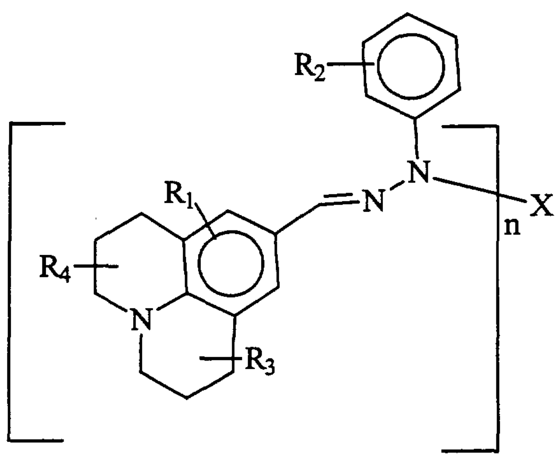

...(3)

where

n is an integer from 2 to 6;

$R_1$, $R_2$, $R_3$, and $R_4$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and

X is a linking group having branched or linear $-(CH_2)_m-$, where m is an integer from 0 to 50, and at least one

methylene group is optionally substituted with a chemical bond, oxygen atom, sulfur atom, a carbonyl group, an urethane group, an urea group, an ester group, a $C_6$-$C_{20}$ aryl group, a $C_5$-$C_{20}$ cycloalkyl group, a cyclosiloxyl group, a heterocyclic group, a $NR_5$ group, a $CHR_6$ group, or a $CR_7R_8$ group where $R_5$, $R_6$, $R_7$, and $R_8$ are, independently, H, a $C_1$-$C_{20}$ alkyl group, or a $C_6$-$C_{20}$ aryl group.

**11.** The charge transport compound of claim 10, wherein the charge transport compound is a compound having formula (4):

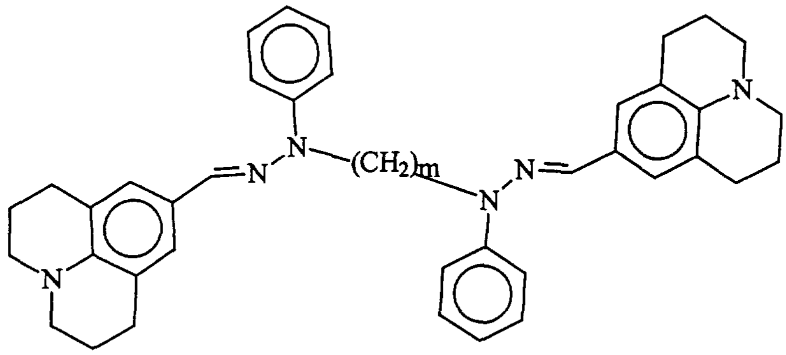

...(4)

where m is an integer from 4 to 10.

**12.** The charge transport compound of any of claims 1 to 7, wherein the charge transport compound is a compound having formula (5):

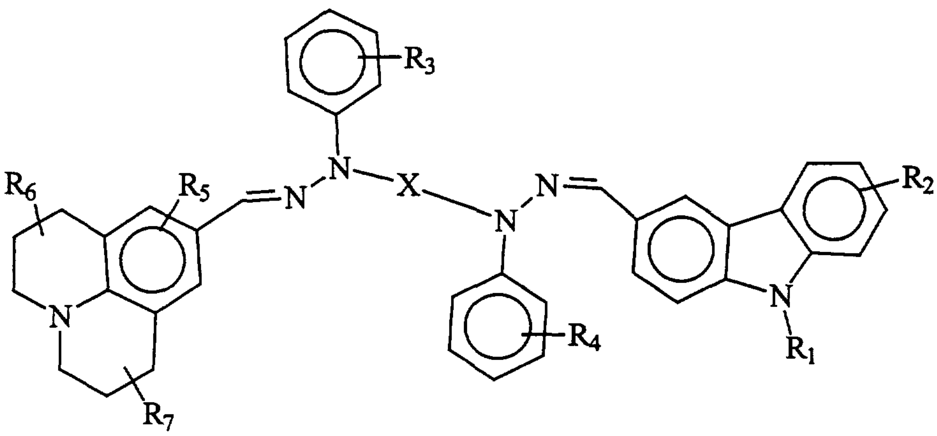

...(5)

where

$R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group;
$R_2$, $R_3$, $R_4$, $R_5$, $R_6$, and $R_7$ are, independently, hydrogen, halogen atom, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and
X is a linking group having a branched or linear $-(CH_2)_m-$, where m is an integer from 0 to 50, and at least one methylene group is optionally substituted with oxygen atom, sulfur atom, a carbonyl group, an urethane group, an urea group, an ester group, a $C_6$-$C_{20}$ aryl group, a heterocyclic group, a $C_5$-$C_{20}$ cycloalkyl group, a cyclosiloxyl group, a $NR_8$ group, a $CHR_9$ group, or a $CR_{10}R_{11}$ group where $R_8$, $R_9$, $R_{10}$, and $R_{11}$, are, independently,

H, a $C_1$-$C_{20}$ alkyl group, or a $C_6$-$C_{20}$ aryl group.

**13.** The charge transport compound of claim 12, wherein the compound having formula (5) is compound (J) having the following formula:

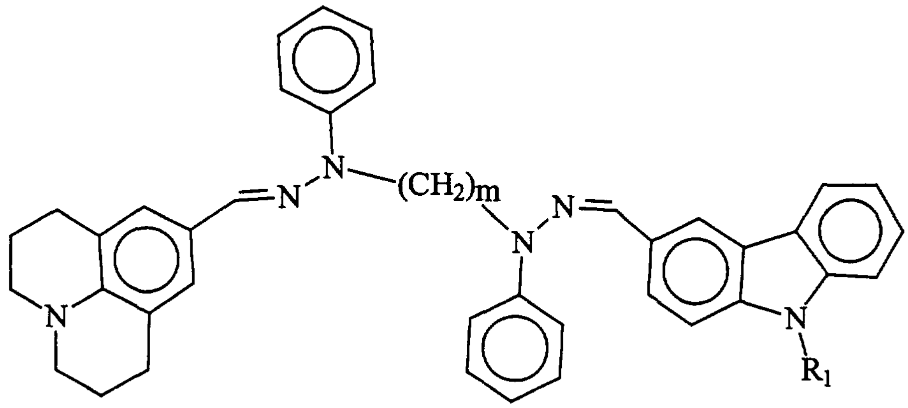

(J)

where m is an integer from 2 to 20, and $R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group.

**14.** The charge transport compound of any of claims 1 to 7, wherein the charge transport compound is a compound having formula (6):

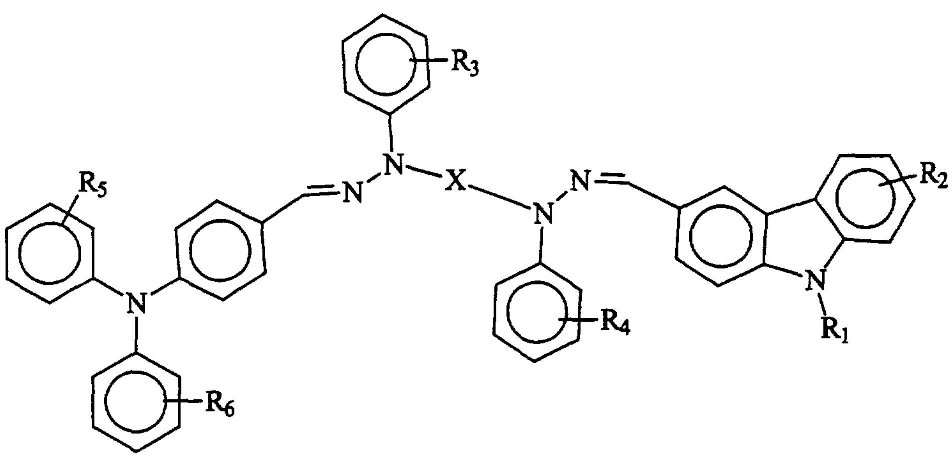

...(6)

where

$R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group;
$R_2$, $R_3$, $R_4$, $R_5$, and $R_6$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and
X is a linking group having branched or linear $-(CH_2)_m-$, where m is an integer from 0 to 50, and at least one methylene group is optionally substituted with oxygen atom, sulfur atom, a carbonyl group, an urethane group,

an urea group, an ester group, a $C_6$-$C_{20}$ aryl group, a heterocyclic group, a $C_5$-$C_{20}$ cycloalkyl group, a cyclosiloxyl group, a $NR_7$ group, a $CHR_8$ group, or a $CR_9R_{10}$ group where $R_7$, $R_8$, $R_9$, and $R_{10}$, are, independently, H, a $C_1$-$C_{20}$ alkyl group, or a $C_6$-$C_{20}$ aryl group.

**15.** The charge transport compound of claim 14, wherein the compound having formula (6) is compound (K) having the following formula:

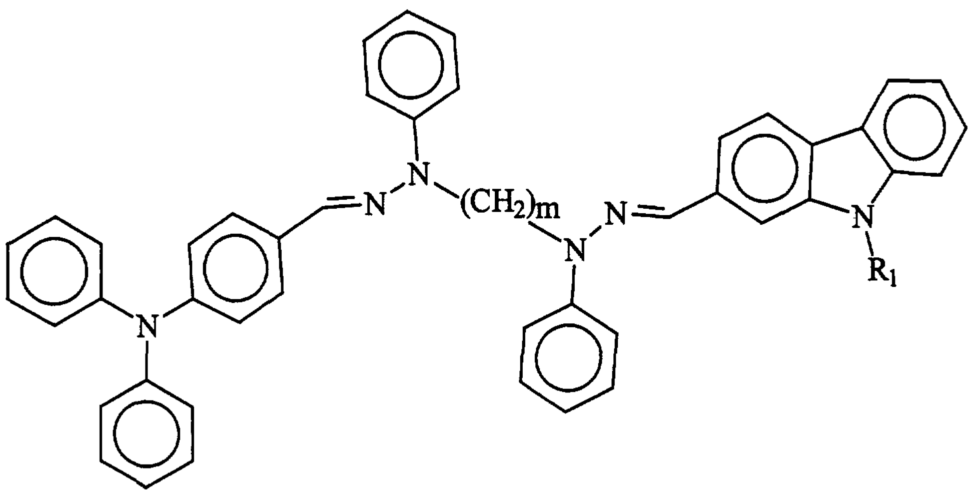

(K)

where m is an integer from 2 to 20, and $R_1$ is hydrogen, a branched or linear $C_1$-$C_{20}$ alkyl group, a branched or linear unsaturated $C_2$-$C_{20}$ hydrocarbon group, a $C_5$-$C_{20}$ cycloalkyl group, or a $C_6$-$C_{20}$ aryl group.

**16.** The charge transport compound of any of claims 1 to 7, wherein the charge transport compound is a compound having formula (7):

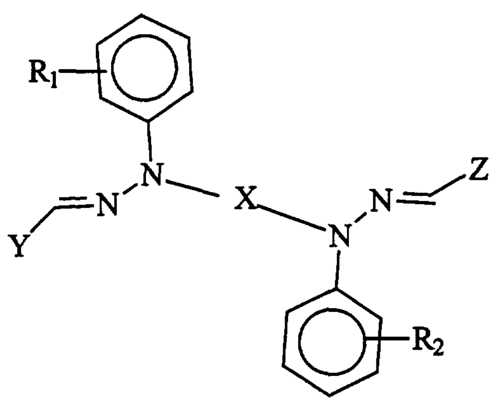

...(7)

wherein

$R_1$ and $R_2$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group;
X is a linking group having branched or linear $-(CH_2)_m-$, where m is an integer from 0 to 50, and at least one methylene group is optionally substituted with a chemical bond, oxygen atom, sulfur atom, a carbonyl group,

an urethane group, an urea group, an ester group, a $C_6$-$C_{20}$ aryl group, a heterocyclic group, a $NR_3$ group, a $CHR_4$ group, or a $CR_5R_6$ group where $R_3$ , $R_4$, $R_5$, and $R_6$ are, independently, H, a $C_1$-$C_{20}$ alkyl group, or a $C_6$-$C_{20}$ aryl group; and

Y and Z are, independently, a carbazole group, a triphenylamine group, a julolidine group, or any of their derivatives.

**17.** The charge transport compound of claim 16, wherein the compound having formula (7) is a compound having formula (8):

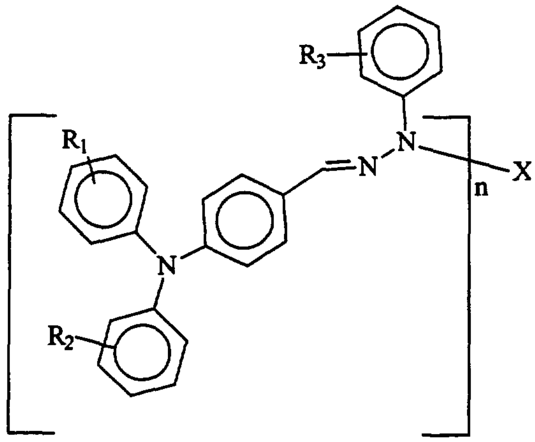

...(8)

where

n is an integer from 2 to 6;

$R_1$, $R_2$, and $R_3$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and

X is a linking group having branched or linear $-(CH_2)_m-$, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a heterocyclic group, a $NR_4$ group, a $CHR_5$ group, or a $CR_6R_7$ group where $R_4$ , $R_5$, $R_6$, and $R_7$ are, independently, H, a $C_1$-$C_{20}$ alkyl group, or a $C_6$-$C_{20}$ aryl group.

**18.** The charge transport compound of claim 17, wherein the compound having formula (8) is compound (M) having the following formula:

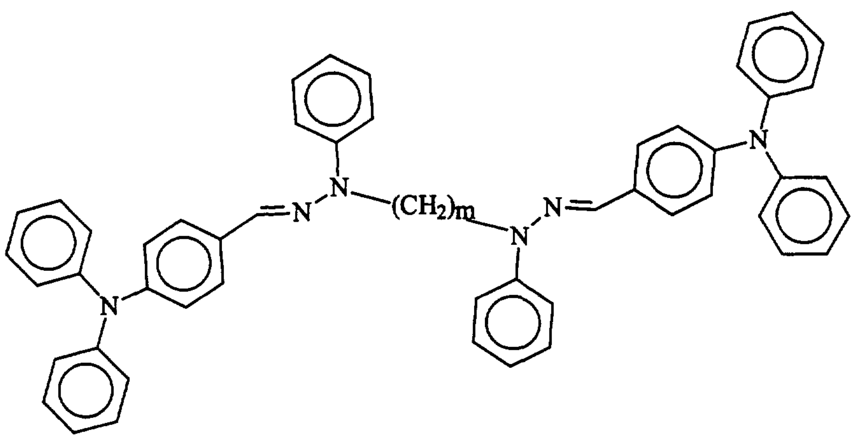

(M)

where m is an integer from 4 to 10.

**19.** The charge transport compound of any of claims 1 to 7, wherein the charge transport compound is a compound having formula (9):

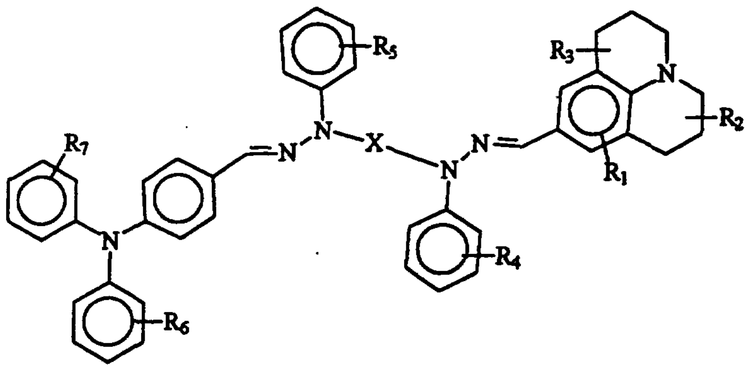

...(9)

where

$R_1$, $R_2$, $R_3$, $R_4$, $R_5$, $R_6$, and $R_7$ are, independently, hydrogen, halogen atom, halide, a hydroxy group, a thiol group, a $C_1$-$C_{20}$ alkoxy group, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, or a $C_6$-$C_{20}$ aryl group; and

X is a linking group having branched or linear $-(CH_2)_m-$, where m is an integer from 0 to 50, and at least one methylene group is optionally substituted with a chemical bond, oxygen atom, sulfur atom, a carbonyl group, an urethane group, an urea group, an ester group, a $C_6$-$C_{20}$ aryl group, a heterocyclic group, a $NR_8$ group, a $CHR_9$ group, or a $CR_{10}R_{11}$ group where $R_8$ , $R_9$, $R_{10}$, and $R_{11}$ are, independently, H, a $C_1$-$C_{20}$ alkyl group, or a $C_6$-$C_{20}$ aryl group.

**20.** The charge transport compound of claim 19, wherein the compound having formula (9) is compound (N) having the following formula:

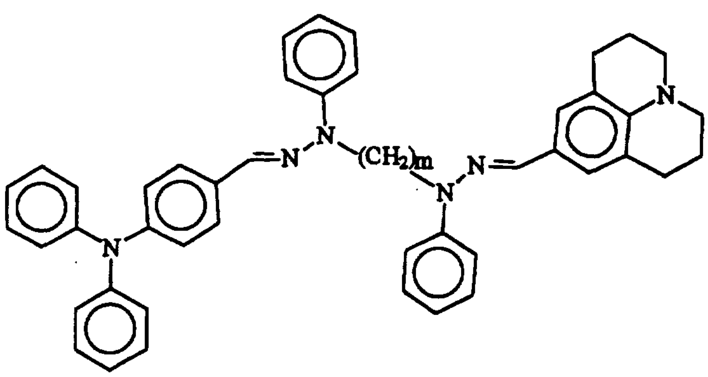

(N)

where m is an integer from 4 to 20.

**21.** The charge transport compound of any of claims 1 to 7, wherein the charge transport compound is a compound having formula (10):

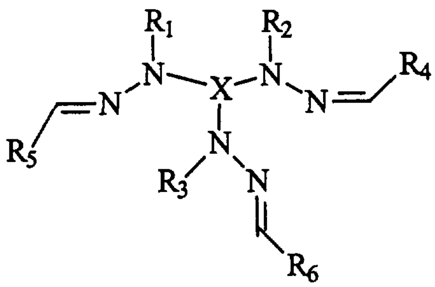

...(10)

where

$R_1$, $R_2$, and $R_3$ are, independently, a branched or linear $C_1$-$C_{20}$ alkyl group, a branched or linear unsaturated $C_2$-$C_{20}$ hydrocarbon group, an ether group, a nitro group, an amino group, a $C_5$-$C_{20}$ cycloalkyl group, a heterocyclic group, or a $C_6$-$C_{20}$ aryl group;
$R_4$, $R_5$, and $R_6$ are, independently, a carbocyclic group selected from the group consisting of triarylamine, diaryl alkylamine, dialkyl arylamine, anthraquinone, diphenoquinone, indane, and fluorenone, or a heterocyclic ring selected from the group consisting of thiazoline, thiazolidine, phenothiazine, oxazoline, imidazoline, imidazolidine, thiazole, oxazole, isoxazole, oxazolidinone, morpholine, imidazole, benzothiazole, benzotriazole, benzoxazole, benzimidazole, naphthothiazole, naphthoxazole, naphthimidazole, quinoline, isoquinoline, quinoxaline, indole, indazole, pyrrole, purine, pyrrolidine, pyridine, piperidine, pyridazine, pyrazoline, pyrimidine, pyrazine, triazole, oxadiazole, tetrazole, urazole, carbazole, julolidine, and thiadiazole, wherein the heterocyclic ring optionally have at least one substituent selected from the group consisting of halogen atom, halide, a $C_1$-$C_{20}$ alkyl group, a $C_1$-$C_{20}$ alkoxy group, a $C_6$-$C_{20}$ aryl group, a $C_6$-$C_{20}$ aryloxy group, a N-substituted amino group, a N-disubstituted amino group, acyl, carbamoyl, sulfamoyl, nitro, cyano, hydroxy, carboxy, sulfonate, oxo, benzo, naptho, indeno, and phosphate; and
X is a linking group having the following formula:

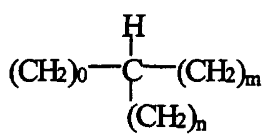

where m, n, and o is an integer from 0 and 50; at least one methylene group is substituted with a chemical bond, oxygen atom, sulfur atom, a carbonyl group, an urethane group, an urea group, an ester group, a $C_6$-$C_{20}$ aryl group, a heterocyclic group, a $NR_5$ group, a $CHR_6$ group, or a $CR_7R_8$ group where $R_5$ , $R_6$, $R_7$, and $R_8$ are, independently, H, a $C_1$-$C_{20}$ alkyl group, or a $C_6$-$C_{20}$ aryl group; and the C-H group is optionally substituted by nitrogen atom, boron atom, metal atom, and a $CR_9$ group where $R_9$ is a $C_1$-$C_{20}$ alkyl group or a $C_6$-$C_{20}$ aryl group.

**22.** The charge transport compound of claim 21, wherein the compound having formula (10) comprises compounds (O) and (P) having the following formulae:

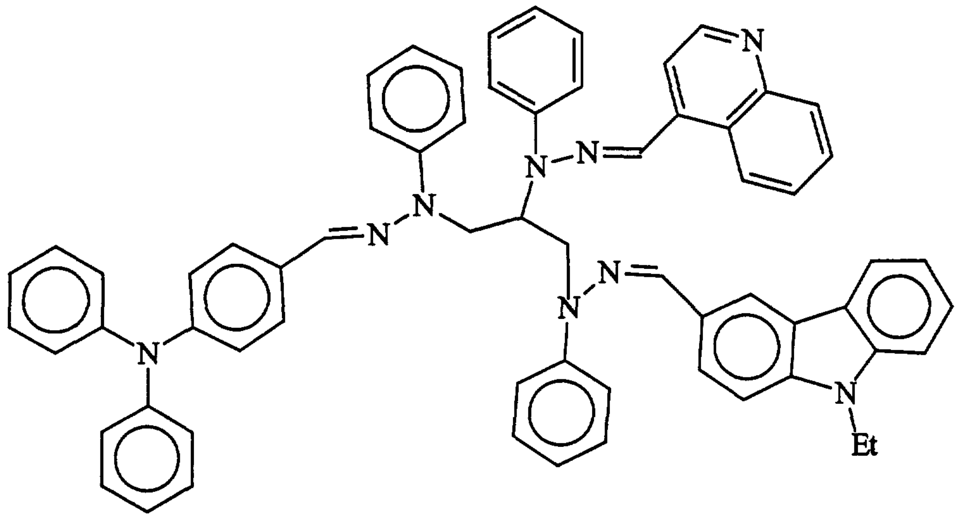

(O)

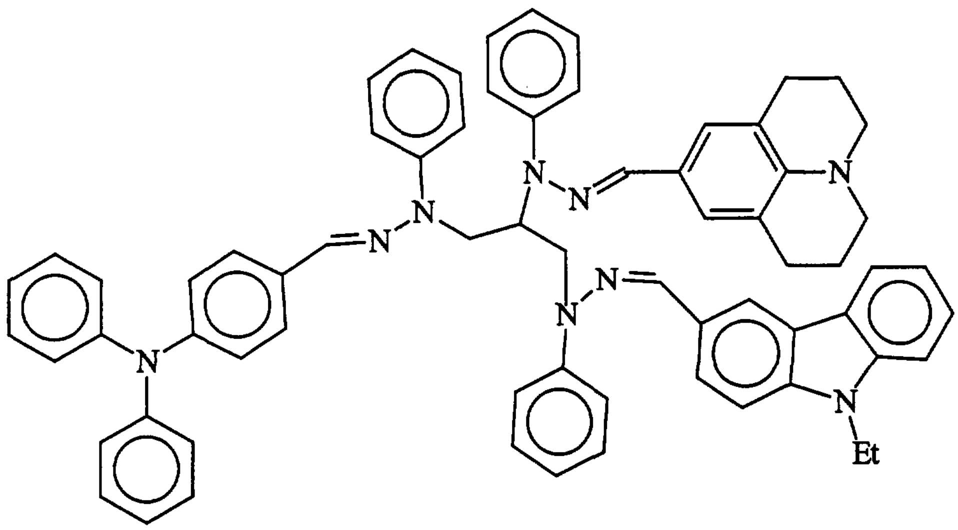

(P)

**23.** An organophotoreceptor comprising a charge transport compound of any of the preceding claims, a charge generating compound, and a conductive substrate.

**24.** The organophotoreceptor of claim 23, being of a flexible belt form.

**25.** The organophotoreceptor of claim 23 or 24, comprising:

a charge transport layer including the charge transport compound and a polymeric binder;
a charge generating layer including the charge generating compound and the polymeric binder; and
the conductive substrate.

**26.** An electrophotographic imaging apparatus comprising:

a plurality of support rollers; and

an organophotoreceptor in the form of a flexible belt wound around the support rollers, the organophotoreceptor including a charge transport compound of any of claims 1 to 22, a charge generating compound, and a conductive substrate.

**27.** The electrophotographic imaging apparatus of claim 26, wherein at least one of the support rollers has a diameter no greater than 40 nm.

**28.** An electrophotographic imaging method comprising:

applying an electric charge to a surface of an organophotoreceptor including a charge transport compound of any of claims 1 to 22, a charge generating compound, and a conductive substrate;
exposing the surface of the organophotoreceptor to radiation to dissipate charge in selected areas and thereby form a pattern of charged and uncharged areas on the surface;
contacting the surface with a liquid toner including a dispersion of colorant particles in an organic liquid to create a toner image; and
transferring the toner image to a substrate.